# EUROPEAN PATENT SPECIFICATION

(11) **EP 1 511 755 B1**
(45) Date of publication and mention of the grant of the patent: **08.03.2006**
(21) Application number: 03730133.0
(22) Date of filing: 28.05.2003
(51) Int. Cl.: C07F 17/00

(54) **TRANSITION METAL COMPOUNDS THEIR PREPARATION AND THEIR USE IN CATALYST SYSTEMS FOR THE POLYMERIZATION AND COPOLYMERIZATION OF OLEFINS**
ÜBERGANGSMETALLVERBINDUNGEN, DEREN HERSTELLUNG UND VERWENDUNG IN KATALYSATORSYSTEMEN FÜR DIE POLYMERISATION UND COPOLYMERISATION VON OLEFINEN
COMPOSES A BASE DE METAUX DE TRANSITION, LEUR ELABORATION ET LEUR UTILISATION DANS DES SYSTEMES CATALYSEURS POUR LA POLYMERISATION ET LA COPOLYMERISATION D'OLEFINES

(30) Priority: 12.06.2002 DE 10226182; 31.07.2002 US 399762 P
(43) Date of publication of application: 09.03.2005
(73) Proprietor: Basell Polyolefine GmbH, 50389 Wesseling (DE)
(72) Inventor: SCHULTE, Jörg, 60487 Frankfurt am Main (DE); SCHOTTEK, Jörg, 60487 Frankfurt am Main (DE); BINGEL, Carsten, 67105 Schifferstadt (DE); OBERHOFF, Markus, 48317 Drensteinfurt (DE)
(86) International application number: PCT/EP2003/005592
(87) International publication number: WO 2003/106470

(56) References cited:
- EP-A- 0 576 970
- EP-B- 0 790 076
- RYABOV A N ET AL: "ZIRCONIUM COMPLEXES WITH CYCLOPENTADIENYL LIGANDS INVOLVING FUSED A THIOPHENE FRAGMENT" ORGANOMETALLICS, ACS, COLUMBUS, OH, US, vol. 21, no. 14, 8 June 2002 (2002-06-08), pages 2842-2855, XP001106373 ISSN: 0276-7333
- DATABASE CROSSFIRE BEILSTEIN [Online] Beilstein Institut zur Förderung der Chemischen Wissenschaften, Frankfurt am Main, DE; Database accession no. BRN 3296411 XP002252207 & COOK ET AL.: J. CHEM. SOC, 1935, pages 1319-1322,
- DATABASE CROSSFIRE BEILSTEIN [Online] Beilstein Institut zur Förderung der Chemischen Wissenschaften, Frankfurt am Main, DE; Database accession no. BRN 3261007 XP002252208 & MAYER; SIEGLITZ: CHEM. BER., vol. 55, 1922, pages 1857-2920,

## Description

The present invention relates to a process for preparing transition metal compounds, in particular ansa-bisindenyl-metallocenes having a specific substitution pattern, the corresponding transition metal compounds themselves and their use in the preparation of catalyst systems and also the use of the catalyst systems in the polymerization and copolymerization of olefins.

Metallocenes can, if appropriate in combination with one or more cocatalysts, be used as catalyst components for the polymerization and copolymerization of olefins. In particular, halogen-containing metallocenes are used as catalyst precursors and are converted, for example, by an aluminoxane into a polymerization-active cationic metallocene complex (EP-A-129368).

The preparation of metallocenes and ansa-metallocenes is known per se (US 4,752,597; US 5,017,714; EP-A-320762; EP-A-416815; EP-A-537686; EP-A-669340; H.H. Brintzinger et al.; Angew. Chem., 107 (1995), 1255; H.H. Brintzinger et al., J. Organomet. Chem. 232 (1982), 233). For this purpose, it is possible, for example, to react cyclopentadienyl-metal compounds with halides of transition metals such as titanium, zirconium and hafnium.

EP-A-0 576 970 describes C₂-symmetric metallocenes having aryl-substituted indenyl derivatives as ligands, the process for preparing them and their use as catalysts. According to this patent application, the metallocene catalysts are formed via a 2-alkyl-4-aryl-1-indanone as intermediate. 2,3-Dialkyl-4-aryl-1-indenes cannot be prepared directly from these indanones.

EP-A-0 629 631 describes C₂-symmetric ansa-bisindenyl-metallocenes substituted with alkyl groups in the positions 4 and 7 of the indenyl ligands and further optionally substituted in the positions 2 and 3 of the indenyl ligand. The catalyst systems obtained therefrom produce polypropylene with a reduced melting point.

EP-A-0 659 757 describes C₁-symmetric metallocenes based on substituted indenyl ligands. The indenyl ligands mentioned there are not substituted in position 3 of the indenyl group.

WO 01/48034 describes ansa-bisindenyl-metallocenes having a combination of different substituents in positions 2 and 4 of the indenyl ligands. The catalyst systems obtained therefrom enable both propylene-ethylene copolymers as rubber phase with a sufficient molar mass and also propylene homopolymers having a sufficiently high melting point for satisfactory stiffness of the matrix to be produced.

Variation of the substitution pattern on the ligand systems of ansa-metallocenes changes the steric environment around the active center and also its electronic structure. This makes it possible to influence, for example, the polymerization behavior of the catalyst constituents and also the final properties of the polymers such as isotacticity, chain length or molar mass as well as the macroscopic material properties of these polymers.

However, particularly in the case of the copolymerization of ethylene/propylene, the catalyst systems of the prior art usually give copolymers having molar masses which are still too low and/or an ethylene content which is still too low. There is therefore a need for suitable catalyst systems which make possible particularly high contents of copolymerized ethylene in the copolymerization of ethylene/propylene combined with high isotacticity of the polypropylene part. There is also a need for catalyst systems which make possible a high molar mass and a high copolymerized ethylene content without deterioration of the molar mass of the copolymer and also an increase in the molar mass of the resulting copolymer compared to the molar mass of the homopolymer. Furthermore, there is a continuing need for simple and highly effective processes for the synthesis of multiply substituted indenyl ligand systems for use in polymerization-active metallocene compounds.

It is an object of the present invention to provide novel C₁- and C₂-symmetric metallocenes as catalysts or catalyst constituents for olefin polymerization which avoid the disadvantages of the prior art and make it possible for the polymerization behavior and the polymer properties to be controlled in a targeted manner.

We have found that this object is achieved by the transition metal compounds as set forth in claim 1, a process for preparing these transition metal compounds as set forth in the independent process claim and their use as catalyst constituent in the (co)polymerization of olefins as set forth in the independent use claim.

Preferred embodiments are given by combining the features of the independent claims with the features of the respective dependent claims.

### Transition metal compound:

It has surprisingly been found that metallocenes having a particular substitution pattern in the 2, 3 and 4 positions of at least one indenyl radical or the sterically corresponding positions of a cyclopentadienyl derivative achieve the abovementioned objects particularly well.

In a first aspect, the present invention accordingly provides transition metal compounds of the formula (I) 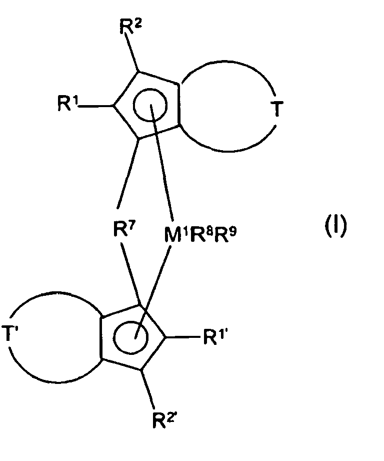
where 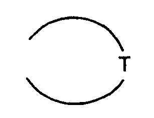
is 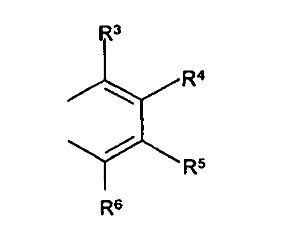
and 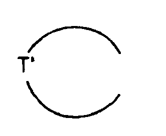
is 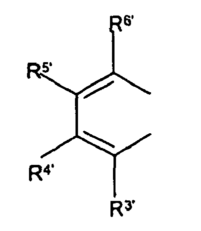
and
- M¹: is titanium, zirconium or hafnium, preferably zirconium;
- R¹,R²: are identical or different and are each a C₁-C₂₀ group such as linear or branched C₁-C₁₈₋alkyl, C₂-C₁₀-alkenyl or C₃-C₁₅-alkylalkenyl; C₆-C₂₀-aryl, C₄-C₁₈-heteroaryl, C₇-C₂₀₋arylalkyl; or fluorinated C₁-C₁₂-alkyl, C₂-C₁₀-alkenyl, C₆-C₂₀-aryl or C₇-C₂₀-arylalkyl,
- R^{1'}, R^{2'}: are identical or different, identical to or different from R¹ or R² and are each hydrogen or a C₁-C₂₀ group such as linear or branched C₁-C₁₈-alkyl, C₂-C₁₀-alkenyl or C₃-C₁₅₋alkylalkenyl; C₆-C₂₀-aryl, C₄-C₁₈-heteroaryl, C₇-C₂₀-arylalkyl; or fluorinated C₁-C₁₂-alkyl, C₂-C₁₀-alkenyl, C₆-C₂₀-aryl or C₇-C₂₀-arylalkyl;
- R³: is a C₆-C₁₈-aryl group or C₄-C₁₈-heteroaryl; or a fluorinated C₆-C₂₀-aryl or C₇-C₂₀₋alkylaryl, where the aryl part of these groups may bear one or more linear or branched C₁-C₁₈-alkyl, C₁-C₁₈-alkoxy, C₂-C₁₀-alkenyl or C₃-C₁₅-alkylalkenyl groups as substituents, or R³ together with R⁴ forms a monocyclic or polycyclic ring system which may in turn be substituted;
- R^{3'}: is hydrogen or a C₁-C₄₀ group such as a C₆-C₁₈-aryl group, C₄-C₁₈-heteroaryl, C₇-C₂₀₋arylalkyl; or fluorinated C₆-C₂₀-aryl or C₇-C₂₀-arylalkyl, where the aryl part of these groups may bear one or more linear or branched C₁-C₁₈-alkyl, C₁-C₁₈-alkoxy, C₂-C₁₀₋alkenyl or C₃-C₁₅-alkylalkenyl groups as substituents, or R^{3'} together with R^{4'} forms a monocyclic or polycyclic ring system which may in turn be substituted;
- R⁴,R⁴': are identical or different and are each hydrogen or a C₁-C₂₀ group such as linear or branched C₁-C₁₈-alkyl, C₂-C₁₀-alkenyl or C₃-C₁₅-alkylalkenyl; C₆-C₂₀-aryl, C₄-C₁₈₋heteroaryl, C₇C₂₀-arylalkyl; or fluorinated C₁-C₁₂-alkyl, C₂-C₁₀-alkenyl, C₆-C₂₀-aryl or C₇₋C₂₀-arylalkyl;
- R⁵,R^{5'},R⁶,R^{6'}: are identical or different and are each hydrogen or a C₁-C₂₀ group such as linear or branched C₁-C₁₈-alkyl, C₂-C₁₀-alkenyl or C₃-C₁₅-alkylalkenyl; C₆-C₂₀-aryl, C₄-C₁₈- heteroaryl, C₇-C₂₀-arylalkyl; or fluorinated C₁-C₁₂-alkyl, C₂-C₁₀-alkenyl, C₆-C₂₀-aryl or C₇₋C₂₀-arylalkyl;
- R⁷: is a bridging structural element between the two indenyl radicals and is selected from the M²R¹⁰R¹¹ group, where M² is silicon, germanium, tin or carbon, preferably silicon, and R¹⁰ and R¹¹ may be identical or different and are each hydrogen or a C₁-C₂₀₋hydrocarbon-containing group such as linear or branched C₁-C₁₀-alkyl, C₆-C₁₄-aryl; trialkylsilyl, in particular trimethylsilyl, triarylsilyl or an alkylarylsilyl group;
- R⁸, R⁹: may be identical or different and are each halogen, linear or branched C₁-C₂₀-alkyl, substituted or unsubstituted phenoxide, or R⁸ and R⁹ may be joined to one another and can form a monocyclic or polycyclic ring system which may in turn be substituted.

Particular preference is also given to metallocenes of the formula (I) in which R^{1'} is not hydrogen, R2' is hydrogen and R^{3'} is C₆-C₂₀-aryl.

According to the present invention, the substituents are further defined as follows:

The term "alkyl" as used in the present context encompasses linear and singly branched or multiply branched saturated hydrocarbons which may also be cyclic. Preference is given to a C₁-C₁₈₋alkyl group such as methyl, ethyl, n-propyl, n-butyl, n-pentyl, n-hexyl, n-heptyl, n-octyl, n-nonyl, n-decyl, cyclopentyl or cyclohexyl, isopropyl, isobutyl, isopentyl, isohexyl, sec-butyl or tert-butyl.

The term "alkenyl" as used in the present context encompasses linear and singly branched or multiply branched hydrocarbons having at least one C-C double bond. In the event of a plurality of C-C double bonds being present, these may be cumulated or conjugated.

The term "alkylalkenyl" as used in the present context encompasses linear and singly branched or multiply branched hydrocarbons having at least one isolated C-C double bond, so that the substituent has both alkyl and alkenyl parts.

The term "aryl" as used in the present context denotes aromatic and fused polyaromatic hydrocarbon substituents which may be substituted by one or more linear or branched C₁-C₁₈-alkyl, C₁₋C₁₈-alkoxy, C₂-C₁₀-alkenyl or C₃-C₁₅-alkylalkenyl groups. Preferred examples of aryl substituents are, in particular, phenyl, 4-methylphenyl, 4-ethylphenyl, 4-propylphenyl, 4-isopropylphenyl, 4-tert-butylphenyl, 4-methoxyphenyl, 1-naphthyl, 9-anthracenyl, 3,5-dimethylphenyl, 3,5-di-tert-butylphenyl or 4-trifluoromethylphenyl.

The term "heteroaryl" as used in the present context denotes aromatic hydrocarbon substituents in which one or more carbon atoms are replaced by nitrogen, phosphorus, oxygen or sulfur atoms or combinations thereof. These may, like the aryl radicals, be substituted by one or more linear or branched C₁-C₁₈-alkyl, C₂-C₁₀-alkenyl or C₃-C₁₅-alkylalkenyl groups. Preferred examples are pyridinyl, pyrazolyl, imidazolyl, oxazolyl, thiazolyl, pyrimidinyl, pyrazinyl and the like, and methyl-, ethyl-, propyl-, isopropyl- and tert-butyl-substituted derivatives thereof.

The term "arylalkyl" as used in the present context denotes aryl-containing substituents whose aryl radical is bound to the indenyl radical via an alkyl chain. Preferred examples are benzyl, substituted benzyl, phenethyl, substituted phenethyl and the like.

The term "fluorinated" means that at least one, preferably more than one and at most all, hydrogen atoms of a substituent are replaced by fluorine atoms. Examples of fluorinated substituents which are preferred for the purposes of the present invention are trifluoromethyl, 2,2,2-trifluoroethyl, pentafluorophenyl, 4-trifluoromethylphenyl, 4-perfluoro-tert-butylphenyl and the like.

The term "bridging structural element" refers to a divalent group which joins the two indenyl radicals to one another via the positions 1 and 1'. Preferred examples are groups having the structure M² R¹⁰R¹¹, where M² is silicon and R¹⁰ and R¹¹ may be identical or different and are each a C₁-C₂₀₋hydrocarbon-containing group such as C₁-C₁₀-alkyl, C₆-C₁₄-aryl, trialkylsilyl, in particular trimethylsilyl, triarylsilyl or an alkylarylsilyl group. Groups which are particularly preferred as bridging structural element are Si(Me)₂, Si(Ph)₂, Si(Me)(Et), Si(Ph)(Me), Si(Ph)(Et), Si(Me)(SiMe₃), Si(Et)₂, where Ph is a substituted or unsubstituted phenyl, Me is methyl and Et is ethyl.

A preferred embodiment of the present invention provides a transition metal compound of the formula (I)
in which:
- M¹: is zirconium;
- R¹,R²: are identical or different and are each a C₁-C₁₂-alkyl group, preferably methyl, ethyl, n-propyl, isopropyl, n-butyl, isobutyl, see-butyl, tert-butyl, cyclopentyl or cyclohexyl, particularly preferably methyl, ethyl or isopropyl;
- R^{1'}, R^{2'}: are identical or different and are each hydrogen, methyl, ethyl, n-propyl, isopropyl, n-butyl, isobutyl, sec-butyl, tert-butyl, cyclopentyl or cyclohexyl, particularly preferably hydrogen, methyl, ethyl or isopropyl, with R^{1'} very particularly preferably being methyl, ethyl or isopropyl and R^{2'} very particularly preferably being hydrogen;
- R³,R^{3'}: are identical or different and are each a C₆-C₁₈-aryl group which may be substituted or unsubstituted, in particular phenyl, 4-methylphenyl, 4-ethylphenyl, 4-propylphenyl, 4-isopropylphenyl, 4-tert-butylphenyl, 4-methoxyphenyl, 1-naphthyl, 9-anthracenyl, 3,5-dimethylphenyl, 3,5-di-tert-butylphenyl or 4-trifluoromethylphenyl, or two radicals R³ together with R⁴ and/or R^{3'} together with R^{4'} may form a monocyclic or polycyclic ring system which may in turn be substituted, in particular a substituted or unsubstituted, preferably unsubstituted, 1,4-buta-1,3-dienylene group, and R^{3'} may also be hydrogen;
- R⁴,R^{4'}: are identical or different and are either hydrogen or R⁴ together with R³ and/or R^{4'} together with R^{3'} may form a monocyclic or polycyclic ring system, with hydrogen being preferred;
- R⁵,R^{5'},R⁶,R^{6'}: are identical or different and are each hydrogen, linear or branched C₁-C₁₈-alkyl, C₂₋C₁₀-alkenyl or C₃-C₁₅-alkylalkenyl; C₆-C₂₀-aryl, C₄-C₁₈-heteroaryl, C₇-C₂₀-arylalkyl; or fluorinated C₁-C₁₂-alkyl, C₂-C₁₀-alkenyl, C₆-C₂₀-alkyl or C₇-C₂₀-arylalkyl, with hydrogen being particularly preferred;
- R⁷: is a bridging structural element SiR¹⁰R¹¹ and R¹⁰ and R¹¹ are identical or different and are each a C₁-C₂₀-hydrocarbon-containing group, with R⁷ particularly preferably being Si(Me)₂, Si(Ph)₂, Si(Et)₂, Si(Me)(Ph), Si(Me)(SiMe₃); and
- R⁸, R⁹: are each chlorine or methyl.

Very particular preference is given to bridged metallocene compounds of the formula (I) in which:
- M¹: is zirconium;
- R¹,R²: are identical or different and are each methyl, ethyl or isopropyl;
- R^{1'},R^{2'}: are identical or different and are each hydrogen, methyl, ethyl or isopropyl, with R^{2'} preferably being hydrogen and R^{1'} particularly preferably being methyl;
- R³,R^{3'}: are identical or different and are each a C₆-C₁₈-aryl group which may be substituted or unsubstituted, in particular phenyl, 4-methylphenyl, 4-ethylphenyl, 4-propylphenyl, 4-isopropylphenyl, 4-tert-butylphenyl, 4-methoxyphenyl, 1-naphthyl, 9-anthracenyl, 3,5-dimethylphenyl, 3,5-di-tert-butylphenyl, 4-trifluoromethylphenyl, C₄-C₁₈-heteroaryl, C₇₋C₂₀-arylalkyl, fluorinated C₆-C₁₈-aryl or fluorinated C₇-C₂₀-arylalkyl, or two radicals R³ together with R⁴ and/or R^{3'} together with R^{4'} may form a monocyclic or polycyclic ring system which may in turn be substituted;
- R⁴,R^{4'}: are identical or different and are either hydrogen or together with R³ and R^{3'} form a monocyclic or polycyclic ring system, with hydrogen and the 4,5-benzindenyl skeleton formed from two radicals being particularly preferred;
- R⁵,R^{5'},R⁶,R^{6'}: are identical or different and are each hydrogen or a C₁-C₂₀ group such as linear or branched C₁-C₁₈-alkyl, C₂-C₁₀-alkenyl or C₃-C₁₅-alkylalkenyl; C₆-C₂₀-aryl, C₄-C₁₈₋heteroaryl, C₇-C₂₀-arylalkyl; or fluorinated C₁-C₁₂-alkyl, C₂-C₁₀-alkenyl, C₆-C₂₀-aryl or C₇₋C₂₀-arylalkyl, with hydrogen being particularly preferred;
- R⁷: is a bridging structural element between the two indenyl radicals, with R⁷ particularly preferably being Si(Me)₂, Si(Ph)₂, Si(Et)₂, Si(Me)(Ph), Si(Me)(SiMe₃); and
- R⁸,R⁹: are each chlorine or methyl.

Nonlimiting examples of very particularly preferred transition metal compounds of the formula (I) are:
dimethylsilanediyl(2,3-dimethyl-4-phenylindenyl)(2-methyl-4-phenylindenyl)zirconium dichloride;
dimethylsilanediyl(2,3-dimethyl-4-phenylindenyl)(2-methyl-4-(1-naphthyl)indenyl)zirconium dichloride;
dimethylsilanediyl(2,3-dimethyl-4-phenylindenyl)(2-methyl-4-(4'-tert-butylphenyl)indenyl)zirconium dichloride;
dimethylsilanediyl(2,3-dimethyl-4-(1-naphthyl)indenyl)(2-methyl-4-(4'-tert-butylphenyl)indenyl)-zirconium dichloride;
dimethylsilanediyl(2,3-dimethyl-4-(4'-tert-butylphenyl)indenyl)(2-methyl-4-(4'-tert-butylphenyl)-indenyl)zirconium dichloride;
dimethylsilanediyl(2,3-dimethyl-4-(4'-tert-butylphenyl)indenyl)(2-methyl-4-(3',5'-di-tert-butylphenyl)indenyl)zirconium dichloride;
dimethylsilanediyl(2,3-dimethyl-4-(4'-tert-butylphenyl)indenyl)(2-ethyl-4-(4'-tert-butylphenyl)indenyl)zirconium dichloride;
dimethylsilanediyl(2,3-dimethyl-4-(4'-tert-butylphenyl)indenyl)(2-isopropyl-4-(4'-tert-butylphenyl)-indenyl)zirconium dichloride;
dimethylsilanediyl(2-methyl-3-ethyl-4-(4'-tert-butylphenyl)indenyl)(2-methyl-4-(4'-tert-butylphenyl)-indenyl)zirconium dichloride;
dimethylsilanediyl(2-methyl-3-ethyl-4-(4'-tert-butylphenyl)indenyl)(2-ethyl-4-(4'-tert-butylphenyl)-indenyl)zirconium dichloride;
dimethylsilanediyl(2-methyt-3-ethyl-4-(4'-tert-butylphenyl)indenyl)(2-isopropyl-4-(4'-tert-butylphenyl)indenyl)zirconium dichloride;
dimethylsilanediyl(2-ethyl-3-methyl-4-(4' -tert-butylphenyl)indenyl)(2-methyl-4-(4'-tert-butylphenyl)-indenyl)zirconium dichloride;
dimethylsilanediyl(2-ethyl-3-methyl-4-(4'-tert-butylphenyl)indenyl)(2-ethyl-4-(4'-tert-butylphenyl)-indenyl)zirconium dichloride;
dimethylsilanediyl(2-isopropyl-3-methyl-4-(4'-tert-butylphenyl)indenyl)(2-methyl-4-(4'-tert-butylphenyl)indenyl)zirconium dichloride;
dimethylsilanediyl(2-isopropyl-3-methyl-4-(4'-tert-butylphenyl)indenyl)(2-ethyl-4-(4'-tert-butylphenyl)indenyl)zirconium dichloride.

The present invention further provides a ligand system of the formula (II) or its double bond isomers, 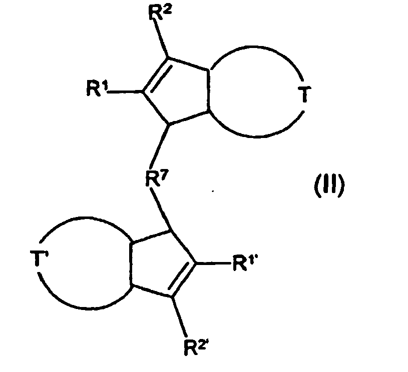
where the variables are as defined for formula (I), including the preferred embodiments.

Compared to most known symmetrically or unsymmetrically substituted metallocenes, the novel metallocenes of the formula (I) give very high molecular weight copolymers in the copolymerization of propylene with other olefins, in particular ethylene. In addition, the copolymers have a molar mass similar to the homopolymer. Particular mention should be made of a high ethylene content in the copolymer and a high polymerization activity in polymerizations carried out under heterogeneous conditions.

Instead of the pure chiral bridged racemic or pseudo-racemic metallocene compounds of the formula (I) it is also possible to use mixtures of the metallocenes of the formula (I) and the corresponding meso or pseudo-meso metallocenes for preparing the catalyst

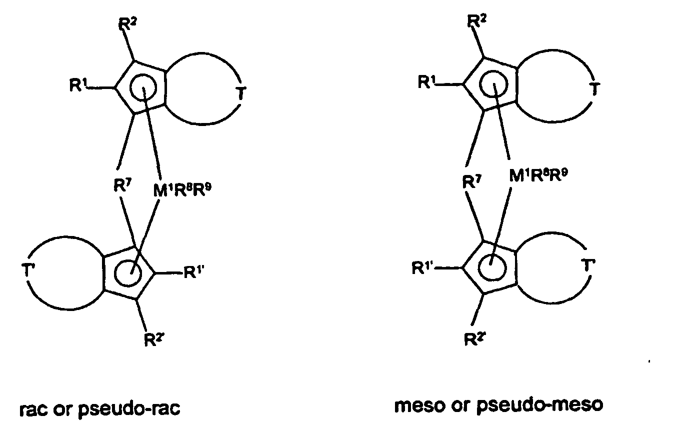

Illustrative but nonrestrictive examples of the metallocenes of the present invention are:
dimethylsilanediylindenyl-L-zirconium dichloride
dimethylsilanediyl-4,5-benzindenyl-L-zirconium dichloride
dimethylsilanediyl-4-phenylindenyl-L-zirconium dichloride
dimethylsilanediyl-4-(4'-tert-butylphenyl)indenyl-L-zirconium dichloride
dimethylsilanediyl-4-(1'-naphthyl)indenyl-L-zirconium dichloride
dimethylsilanediyl-4-(3',5'-di-tert-butylphenyl)indenyl-L-zirconium dichloride
dimethylsilanediyl(2-methylindenyl)-L-zirconium dichloride
dimethylsilanediyl(2-methyl-4,5-benzindenyl)-L-zirconium dichloride
dimethylsilanediyl(2-methyl-4-phenylindenyl)-L-zinconium dichloride
dimethylsilanediyl(2-methyl-4-(4'-tert-butylphenyl)indenyl)-L-zirconium dichloride
dimethylsilanediyl(2-methyl-4-(1'-naphthyl)indenyl)-L-zirconium dichloride
dimethylsilanediyl(2-methyl-4-(3'.S'-di-tert-butylphenyl)indenyl)-L-zirconium dichloride
dimethylsilanediyl(2-ethylindenyl)-L-zirconium dichloride
dimethylsilanediyl(2-ethyl-4-phenylindenyl)-L-zirconium dichloride
dimethylsilanediyl(2-ethyl-4-(4'-tert-butylphenyl)indenyl)-L-zirconium dichloride
dimethylsilanediyl(2-ethyl-4-(1'-naphthyl)indenyl)-L-zirconium dichloride
dimethylsilanediyl(2-ethyl-4-(3',5'-di-tert-butylphenyl)indenyl)-L-zirconium dichloride
dimethylsilanediyl(2-ethyl-4-(4'-trifluoromethylphenyl)indenyl)-L-zirconium dichloride
dimethylsilanediyl(2-propyl-4-phenylindenyl)-L-zirconium dichloride
dimethylsilanediyl(2-propyl-4-(1'-naphthyl)indenyl)-L-zirconium dichloride
dimethylsilanediyl(2-propyl-4-(3',5'-di-tert-butylphenyl)indenyl)-L-zirconium dichloride
dimethylsilanediyl(2-isopropylindenyl)-L-zirconium dichloride
dimethylsilanediyl(2-isopropyl-4-phenylindenyl)-L-zirconium dichloride
dimethylsilanediyl(2-isopropyl-4-(4' -tert-butylphenyl)indenyl)-L-zirconium dichloride
dimethylsilanediyl(2-isopropyl-4-(1'-naphthyl)indenyl)-L-zirconium dichloride
dimethylsilanediyl(2-isopropyl-4-(3',5'-di-tert-butylphenyl)indenyl)-L-zirconium dichloride
dimethylsilanediyl(2-isopropyl-4-(4'-trifluoromethylphenyl)indenyl)-L-zirconium dichloride
dimethylsilanediyl(2-isobutyJ-4-phenylindenyl)-L-zirconium dichloride
dimethylsilanediyl(2-isobutyJ-4-(4' -tert-butylphenyl)indenyl)-L-zirconium dichloride
dimethylsilanediyl(2-isobutyl-4-(1'-naphthyl)indenyl)-L-zirconium dichloride
dimethylsilanediyl(2-isobutyl-4-(3',5'-di-tert-butylphenyl)indenyl)-L-zirconium dichloride
dimethylsilanediyl(2-butyl-4-phenylindenyl)-L-zirconium dichloride
dimethylsilanediyl(2-butyl-4-(4'-tert-butylphenyl)indenyl)-L-zirconium dichloride
dimethylsilanediyl(2-butyl-4-(3',5'-di-tert-butylphenyl)indenyl)-L-zirconium dichloride
dimethylsilanediyl (2-cyclopentyl-4-(4'-tert-butylphenyl)indenyl)-L-zirconium dichloride
dimethylsilanediyl(2-cyclopentyl-4-(3',5'-di-tert-butylphenyl)indenyl)-L-zirconium dichloride
dimethylsilanediyl(2-cyclohexyl-4-(4'-tert-butylphenyl)indenyl)-L-zirconium dichloride
dimethylsilanediyl(2-cyclohexyl-4-(3',5'-di-tert-butylphenyl)indenyl)-L-zirconium dichloride
dimethylsilanediyl(3-methylindenyl)-L-zirconium dichloride
dimethylsilanediyl(3-methyl-4-phenylindenyl)-L-zirconium dichloride
dimethylsilanediyl(3-methyl-4-(4'-tert-butylphenyl)indenyl)-L-zirconium dichloride
dimethylsilanediyl(3-methyl-4-(3',5'-di-tert-butylphenyl)indenyl)-L-zirconium dichloride
dimethylsilanediyl(2,3-dimethylindenyl)-L-zirconium dichloride
dimethylsilanediyl(2,3-dimethyl-4,5-benzindenyl)-L-zirconium dichloride
dimethylsilanediyl(2,3-dimethyl-4-phenylindenyl)-L-zirconium dichloride
dimethylsilanediyl(2,3-dimethyl-4-(4'-tert-butylphenyl)indenyl)-L-zirconium dichloride
dimethylsilanediyl(2,3-dimethyl-4-(1' -naphthyl)indenyl)-L-zirconium dichloride
dimethylsilanediyl(2,3-dimethyl-4-(3',5'-di-tert-butylphenyl)indenyl)-L-zirconium dichloride
dimethylsilanediyl(2,3-dimethyl-4-(4' -trifluoromethylphenyl)indenyl)-L-zirconium dichloride
dimethylsilanediyl(2-methyl-3-ethyl-4-phenylindenyl)-L-zirconium dichloride
dimethylsilanediyl(2-methyl-3-ethyl-4-(4'-tert-butylphenyl)indenyl)-L-zirconium dichloride
dimethylsilanediyl(2-methyl-3-ethyl-4-(4'-methoxyphenyl)indenyl)-L-zirconium dichloride
dimethylsilanediyl(2-methyl-3-ethyl-4-(1'-naphthyl)indenyl)-L-zirconium dichloride
dimethylsilanediyl(2-methyl-3-ethyl-4-(9'-anthracenyl)indenyl)-L-zirconium dichloride
dimethylsilanediyl(2-methyl-3-ethyl-4-(3',5'-di-tert-butylphenyl)indenyl)-L-zirconium dichloride
dimethylsilanediyl(2-ethyl-3-methyl-4-phenylindenyl)-L-zirconium dichloride
dimethylsilanediyl(2-ethyl-3-methyl-4-(4'-tert-butylphenyl)indenyl)-L-zirconium dichloride
dimethylsilanediyl(2-ethyl-3-methyl-4-(3',5'-di-tert-butylphenyl)indenyl)-L-zirconium dichloride
dimethylsilanediyl(2-isopropyl-3-methyl-4-phenylindenyl)-L-zirconium dichloride
dimethylsilanediyl(2-isopropyl-3-methyl-4-(1'-naphthyl)indenyl)-L-zirconium dichloride
dimethylsilanediyl(2-isopropyl-3-methyl-4-(3',5'-di-tert-butylphenyl)indenyl)-L-zirconium dichloride
dimethylsilanediyl(2-isobutyl-3-methyl-4-phenylindenyl)-L-zirconium dichloride
dimethylsilanediyl(2-isobutyl-3-methyl-4-(4'-tert-butylphenyl)indenyl)-L-zirconium dichloride
dimethylsilanediyl(2-isobutyl-3-methyl-4-(1'-naphthyl)indenyl)-L-zirconium dichloride
dimethylsilanediyl(2-isobutyl-3-methyl-4-(3',5'-di-tert-butylphenyl)indenyl)-L-zirconium dichloride,

where L represents one of the following substructures:
(2,3-dimethyl-4,5-benzindenyl); (2,3-dimethyl-4-phenylindenyl): (2,3-dimethyl-4-(4'-methylphenyl)-indenyl); (2,3-dimethyl-4-(4'-tert-butylphenyl)indenyl); (2,3-dimethyl-4-(3',5'-di-tert-butylphenyl)-indenyl); (2,3-dimethyl-4-(4'-trifluoromethylphenyl)indenyl); (2,3-diethylindenyl); (2,3-diethyl-4-phenylindenyl); (2,3-diethyl-4-(4'-tert-butylphenyl)indenyl); (2,3-diethyl-4-(1'-naphthyl)indenyl); (2,3-diethyl-4-(3',5'-di-tert-butylphenyl)indenyl); (2,3-diethyl-4-(4'-trifluoromethylphenyl)indenyl); (2,3-dipropyl-4-phenylindenyl); (2,3-dipropyl-4-(4'-tert-butylphenyl)indenyl); (2, 3-dipropyl-4-(1'-naphthyl)indenyl); (2,3-dipropyl-4-(3',5'-di-tert-butylphenyl)indenyl); (2-methyl-3-ethylindenyl); (2-methyl-3-ethyl-4-phenylindenyl); (2-methyl-3-ethyl-4-(4'-ethylphenyl)indenyl); (2-methyl-3-ethyl-4-(4'-tert-butylphenyl)indenyl); (2-methyl-3-ethyl-4-(1'-naphthyl)indenyl); (2-methyl-3-ethyl-4-(3',5'-di-tert-butylphenyl)indenyl); (2-methyl-3-ethyl-4-(4'-trifluoromethylphenyl)indenyl); (2-methyl-3-isopropylindenyl); (2-methyl-3-isopropyl-4-phenylindenyl); (2-methyl-3-isopropyl-4-(4'-methylphenyl)indenyl); (2-methyl-3-isopropyl-4-(4'-tert-butylphenyl)indenyl); (2-methyl-3-isopropyl-4-(1'-naphthyl)indenyl); (2-methyl-3-isopropyl-4-(3',5'-di-tert-butylphenyl)indenyl); (2-ethyl-3-methyl-4-phenylindenyl); (2-ethyl-3-methyl-4-(4'-tert-butylphenyl)indenyl); (2-ethyl-3-methyl-4-(1'-naphthyl)indenyl); (2-ethyl-3-methyl-4-(3',5'-di-tert-butylphenyl)indenyl).

Preference is also given to the corresponding dimethylzirconium compounds, the corresponding η⁴-butadienezirconium compounds and metallocenes of the formula (I) having zirconium fragments as described in WO 00/31090, and also the corresponding titanium and hafnium compounds.

Since, in particular, the interplay of the steric effects of the radicals R¹, R² and R³ together with the radicals R¹, R² and R³ is important for the polymerization properties of the novel transition metal compounds of the formula (I), the indenyl skeleton can in principle also be replaced by a structurally similar, in particular heteroatom-containing, bicyclic or polycyclic hydrocarbon skeleton (cf. WO 98/22486) which may contain, for example, sulfur, nitrogen, oxygen or phosphorus, preferably nitrogen or sulfur, e.g. a correspondingly substituted cyclopenta[2,3-b]thiophen-6-yl or cyclopenta[2,3-b]pyrrol-4-yl skeleton: 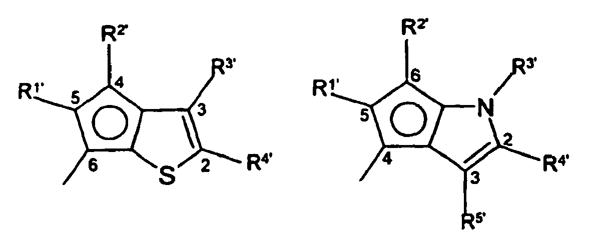
or

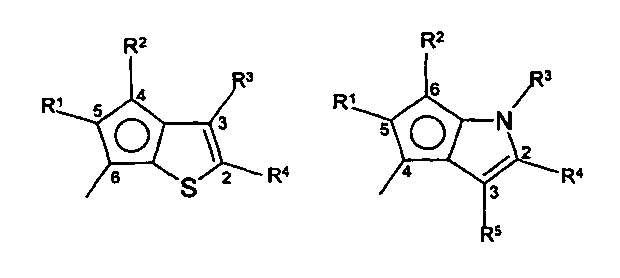

Nonrestrictive examples of such heteroatom-containing radicals are 5-methylcyclopenta[2,3-b]thiophen-6-yl, 5-ethylcyclopenta[2,3-b]thiophen-6-yl, 5-isopropylcyclopenta[2,3-b]thiophen-6-yl, 2,3,5-trimethylcyclopenta[2,3-b]thiophen-6-yl, 3,5-dimethylcyclopenta[2,3-b]thiophen-6-yl, 2,5-dimethyl-3-phenylcyclopenta[2,3-b]thiophen-6-yl, 2,5-dimethyl-3-(p-t-butylphenyl)cyclopenta[2,3-b]thiophen-6-yl, 5-isopropyl-2-methyl-3-(p-t-butylphenyl)cyclopenta[2,3-b]thiophen-6-yl, 2,3,5-trimethyl-3-(p-t-butylphenyl)cyclopenta[2,3-b]thiophen-6-yl, 1,5-dimethylcyclopenta[2,3-b]pyrronl-4-yl, 1,2,3,5-tetramethylcyclopenta[2,3-b]pyrrol-4-yl, 2,5-dimethyl-1-phenylcyclopenta[2,3-b]pyrrol-4-yl, 2,5-dimethyl-1-(p-t-butylphenyl)cyclopenta[2,3-b]pyrrol-4-yl, 5-isopropyl-2-methyl-1-(p-t-butylphenyl)cyclopenta[2,3-b]pyrrol-4-yl and 2,5,6-trimethyl-1-phenylcyclopenta[2,3-b]pyrrol-4-yl.

### Synthesis of the transition metal compounds:

We have surprisingly found a synthetic route by means of which it is possible to prepare metallocenes which have a specific substitution pattern and achieve the objects of the invention particularly well. Selected ansa-bisindenyl-metallocenes, in particular those which bear at least one, in particular exactly one, indenyl ligand which bears substituents different from hydrogen in the positions 2, 3 and 4, achieve these objects particularly well. According to the present invention, it is possible to achieve a particularly high copolymerized ethylene content in the copolymerization of ethylene/propylene combined with a high isotacticity of the polypropylene part by means of a particular combination of different indenes.

The synthesis of the metallocenes of the present invention is in principle carried out according to the following simplified scheme: 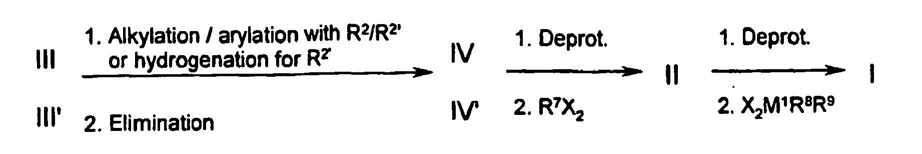
where X is Cl, Br, I, O-tosyl and all other constituents and substituents are as described for formula (I), and where III, III', IV and VI' are the following structures:

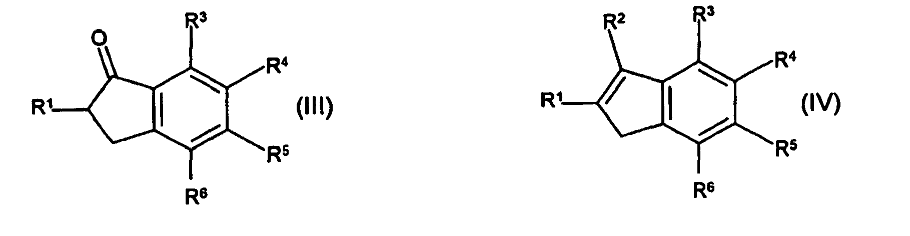

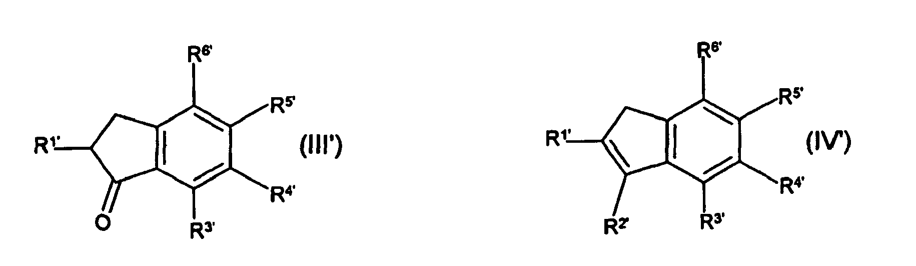

The present invention thus also provides a process for preparing ansa-metallocenes of the formula (I) which comprises the following steps:
a) reaction of a 1-indanone of the formula (III) or (III') with an organometallic compound M³R²ₘHalₙ or M³R^{2'}ₘHalₙ and subsequent elimination to form the substituted indene of the formula (IV) or (IV'),
   where the variables R¹, R^{1'}, R²**,** R^{2'}, R³, R^{3'}, R⁴, R^{4'}, R⁵, R^{5'}, R⁶ and R^{6'} are as defined for formula (1), M³ is an alkali metal, an alkaline earth metal, aluminum or titanium, Hal is halogen, m is an integer and is equal to or greater than 1 and the sum of m+n corresponds to the valence of M³;
   deprotonation of the substituted indene of the formula (IV) or (IV') and subsequent reaction of the deprotonated indene with compounds of the type R⁷X₂ to form compounds of the formula (V) or (V') or their double bond isomers, 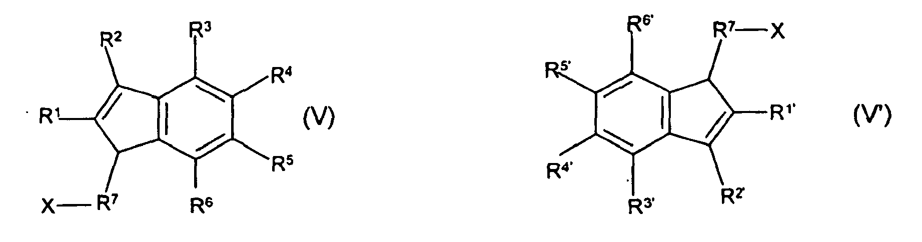
   where X is Cl, Br, I or O-tosyl and R⁷ is as defined for formula (I);
c) reaction of the compound of the formula (V) or (V') with a further deprotonated indene which has been obtained by deprotonation of (IV) or (IV') to form the ligand system of the formula (IIa) or its double bond isomers, 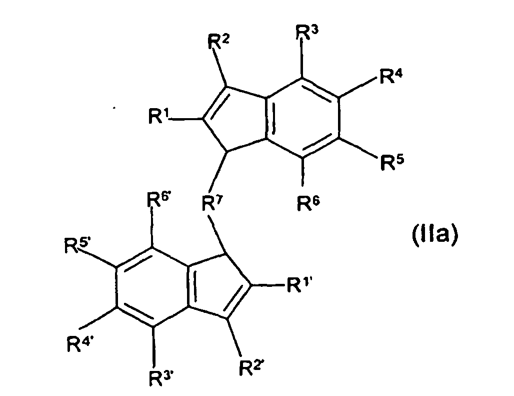
d) deprotonation of the ligand system of the formula (IIa) or its double bond isomers and reaction with compounds of the type X₂M¹R⁸R⁹ to give the ansa-metallocene of the formula (I), where X is as defined for formula (V) and M¹, R⁸ and R⁹ are as defined for formula (I).

This gives the corresponding transition metal compounds of the formula (I) 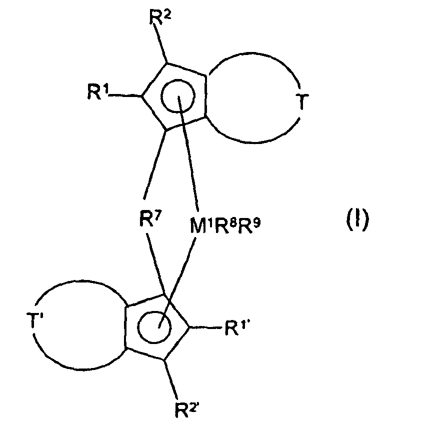
where 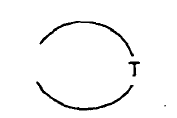
is 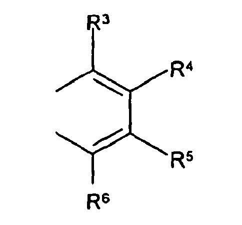
and 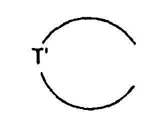
is 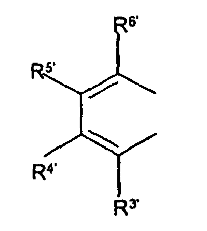
and the substituents R³, R⁴, R⁵, R⁶, R^{3'}, R^{4'}, R^{5'} and R^{6'} are as defined above for formula (I).

The process of the present invention is preferably employed for preparing metallocenes of the formula (I) using compounds whose substituents are as follows:
- M¹: is zirconium;
- R¹, R²: are identical or different and are each a C₁-C₁₂-alkyl group, preferably methyl, ethyl, n-propyl, isopropyl, n-butyl, tert-butyl, isobutyl, cyclopentyl or cyclohexyl, particularly preferably methyl, ethyl or isopropyl;
- R1',R2': are identical or different and are each hydrogen, methyl, ethyl, n-propyl, isopropyl, n-butyl, isobutyl, tert-butyl, cyclopentyl or cyclohexyl, particularly preferably hydrogen, methyl, ethyl or isopropyl, with R^{1'} very particularly preferably being methyl, ethyl or isopropyl and R^{2'} very particularly preferably being hydrogen;
- R³, R^{3'}: are identical or different and are each a C₆-C₁₈-aryl group which may be substituted or unsubstituted, in particular phenyl, 4-methylphenyl, 4-ethylphenyl, 4-propylphenyl, 4-isopropylphenyl, 4-tert-butylphenyl, 4-methoxyphenyl, 1-naphthyl, 9-anthracenyl, 3.5-dimethylphenyl, 3,5-di-tert-butylphenyl or 4-trifluoromethylphenyl; or two radicals R³ together with R⁴ and/or R^{3'} together with R^{4'} may form a monocyclic or polycyclic ring system which may in turn be substituted, in particular a substituted or unsubstituted, preferably unsubstituted, 1,4-buta-1,3-dienylene group, and R^{3'} may also be hydrogen;
- R⁴, R^{4'}: are identical or different and are either hydrogen or together with R³ or R^{3'} form a monocyclic or polycyclic ring system, with hydrogen being particularly preferred;
- R⁵,R^{5'},R⁶,R^{6'}: are identical or different and are each hydrogen or a C₁-C₂₀ group such as linear or branched C₁-C₁₈-alkyl, C₂-C₁₀-alkenyl or C₃-C₁₅-alkylalkenyl; C₆-C₂₀-aryl, C₄-C₁₈₋heteroaryl, C₇-C₂₀-arylalkyl; or fluorinated C₁-C₁₂-alkyl, C₂-C₁₀-alkenyl, C₆-C₂₀-aryl or C₇₋C₂₀-arylalkyl, with hydrogen being particularly preferred;
- R⁷: is a bridging structural element between the two indenyl radicals, with R⁷ particularly preferably being Si(Me)₂, Si(Ph)₂, Si(Et)₂, Si(Me)(Ph), Si(Me)(SiMe₃); and
- R⁸,R⁹: are each chlorine or methyl.

The process of the present invention is particularly preferably employed for preparing metallocenes of the formula (I) using compounds whose substituents are as follows:
- M¹: is zirconium;
- R¹, R²: are identical or different and are each methyl, ethyl or isopropyl;
- R^{1'}, R^{2'}: are identical or different and are each hydrogen, methyl, ethyl or isopropyl, with R^{2'} preferably being hydrogen and R^{1'} particularly preferably being methyl;

and all other substituents are as defined above.

The substituted 1-indanones of the formula (III) or (III') are obtainable in a simple manner by synthetic methods known from the prior art, for example the process described in WO 98/40331.

In the process of the present invention, the addition of the radicals R² or R^{2'} onto the carbon atom of the keto group is achieved by reaction of the cyclic ketone with a suitable organometallic compound M³R²ₘHalₙ or M³R^{2'}ₘHalₙ, where M³ is an alkali metal, an alkaline earth metal, aluminum or titanium and Hal is halogen, e.g. a Grignard, lithium, titanium or aluminum reagent. These organometallic compounds are likewise obtainable in a simple manner by standard methods of the prior art or can be purchased commercially. The synthesis of appropriate Grignard reagents is described, for example, in Holm, Torkil, J. Chem. Soc. Perkin Trans. 2, 1981, 464-467, and also in March, Advanced Organic Chemistry, 4^{th} Edition 1992, and the references cited therein. A person skilled in the art will choose appropriate organometallic compounds depending on the specific substitution patterns and reactivities of the indanone compounds to be reacted.

After the reaction with the suitable organometallic compound M³R²ₘHalₙ or M³R^{2'}ₘHalₙ, an elimination reaction is carried out to form the double bond in the 5-membered ring. This can, for example, be induced by means of a suitable dilute or undiluted acid, e.g. hydrochloric acid, sulfuric acid, phosphoric acid or an organic acid such as formic acid, acetic acid, citric acid and the like; preference is in most cases given to approximately 6 N hydrochloric acid.

This gives a substituted indene of the formula (IV) or (IV) which, after deprotonation on the methylene carbon of the 5-membered ring, is reacted with a reagent R⁷X₂, in the simplest case a dialkyldichlorosilane, for example, to form a compound of the formula (V) or (V'). The deprotonation is carried out using suitable bases such as n-butyllithium, tert-butyllithium, methyllithium, potassium hydride, dibutylmagnesium or the like. Appropriate process steps are known from the prior art and are described, for example, in WO 01/48034.

The compounds of the formula (V) or (V') are subsequently reacted with deprotonated (III) or (III') to form the corresponding ligand system (IIa). According to the present invention, this gives ligands of the formula (IIa) which preferably bear at least one indenyl group which is substituted in each of the 2, 3 and 4 positions by a radical different from hydrogen.

The ligands (IIa) obtained in this way or (II) obtained analogously are in turn converted by deprotonation and subsequent reaction with compounds of the type X₂M¹F⁸R⁹ into the corresponding C1- or C2-symmetric, preferably C1-symmetric, ansa-metallocenes of the formula (I). The procedures for synthesizing the complexes are known standard methods of the prior art. The corresponding heterocyclic systems are obtained analogously using the corresponding heteroatom-containing hydrocarbon compounds, as also described in WO 98/22486 and as indicated above.

The process of the present invention will once again be illustrated below by means of a specific and nonrestrictive example:

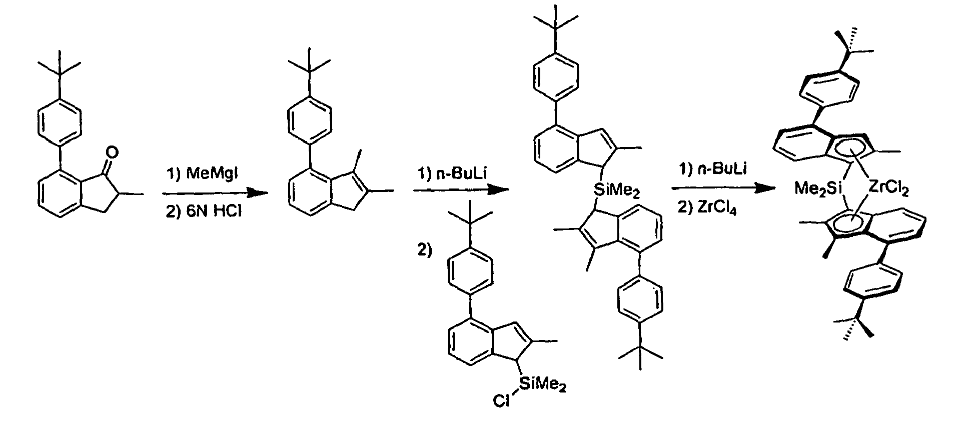

Here, a substituted 1-indanone, e.g. the depicted 7-(4'-t-butylphenyl)-2-methyl-1-indanone which can be prepared as described in WO 98/40331, is reacted with an alkylating organometallic compound, e.g. a Grignard, lithium, titanium or aluminum reagent. Acid-induced elimination gives a 2,3,4-trisubstituted indene which is reacted with a silylated indene which can be prepared as described in WO 01/48034 to form the ligand which can be converted by standard methods into the complex.

The present invention also provides indenes of the formula (IV) or the double bond isomers thereof 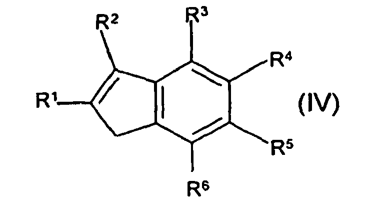
where
- R¹, R²: are identical or different and are each a C₁-C₂₀ group;
- R³: is a C₆-C₁₈-aryl group or C₄-C₁₈-heteroaryl; or a fluorinated C₆-C₂₀-aryl or C₇-C₂₀₋alkylaryl, where the aryl part of these groups may bear one or more linear or branched C₁-C₁₈-alkyl, C₁-C₁₈-alkoxy, C₂-C₁₀-alkenyl or C₃-C₁₅-alkylalkenyl groups as substituents;
- R⁴: is hydrogen or a C₁-C₂₀ group;
- R⁵, R⁶: are identical or different and are each hydrogen or a C₁-C₂₀ group.

The novel metallocenes of the formula (I) are highly active catalyst components for the homopolymerization and copolymerization of olefins. Depending on the substitution pattern of the ligands, the metallocenes can be obtained as a mixture of isomers. The metallocenes are preferably used in diastereomerically pure form for the polymerization.

Preference is given to using the racemic or pseudo-racemic metallocenes of the formula (I), but the use of racemic or pseudo-rac-enriched (pseudo-)rac/(pseudo-) meso mixtures can also be appropriate.

The novel metallocenes of the formula (I) are particularly suitable as constituents of catalyst systems for preparing polyolefins by polymerization of at least one olefin in the presence of a catalyst comprising at least one cocatalyst and at least one metallocene.

### Cocatalysts

The present invention therefore also provides a catalyst system comprising at least one metallocene of the formula (I) (component A) as organometallic transition metal compound and at least one cocatalyst (component B).

Together with the novel metallocene of the formula (I), the cocatalyst forms a polymerization-active catalyst system in which the cocatalyst serves as cation-forming compound.

Suitable cation-forming compounds (components B) which are able to react with a novel organometallic transition metal compound to convert it into a cationic compound are, for example, compounds such as an aluminoxane, a strong uncharged Lewis acid, an ionic compound having a Lewis-acid cation or an ionic compound containing a Brönsted acid as cation. In the case of metallocene complexes as organometallic transition metal compound, the cation-forming compounds are frequently also referred to as compounds capable of forming metallocenium ions.

As aluminoxanes, it is possible to use, for example, the compounds described in WO 00/31090. Particularly useful compounds are open-chain or cyclic aluminoxane compounds of the formula (VI) or (VII) 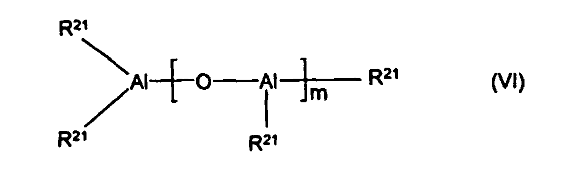 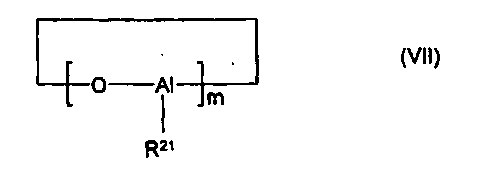
where
- R²¹: is a C₁-C₄-alkyl group, preferably a methyl or ethyl group, and m is an integer from 5 to 30, preferably from 10 to 25.

These oligomeric aluminoxane compounds are usually prepared by reacting a solution of trialkylaluminum with water. The oligomeric aluminoxane compounds obtained in this way are generally in the form of mixtures of both linear and cyclic chain molecules of various lengths, so that m is to be regarded as a mean. The aluminoxane compounds can also be present in admixture with other metal alkyls, preferably aluminum alkyls.

In place of the aluminoxane compounds of the formulae (VI) or (VII), modified aluminoxanes in which some of the hydrocarbon radicals or hydrogen atoms are replaced by alkoxy, aryloxy, siloxy or amide groups can also be used as component B).

It has been found to be advantageous to use the novel organometallic transition metal compound and the aluminoxane compounds in such amounts that the atomic ratio of aluminum from the aluminoxane compounds to the transition metal from the organometallic transition metal compound is in the range from 10:1 to 1000:1, preferably from 20:1 to 500:1 and in particular in the range from 30:1 to 400:1.

As strong, uncharged Lewis acids, preference is given to compounds of the formula (VIII)

M⁴X¹X²X³ (VIII)

where
- M⁴: is an element of group 13 of the Periodic Table of the Elements, in particular B, AI or Ga, preferably B,
- X¹,X² and X³: are each hydrogen, C₁-C₁₀-alkyl, C₆-C₁₅-aryl, alkylaryl, arylalkyl, haloalkyl or haloaryl each having from 1 to 10 carbon atoms in the alkyl radical and from 6 to 20 carbon atoms in the aryl radical or fluorine, chlorine, bromine or iodine, in particular haloaryl, preferably pentafluorophenyl.

Further examples of strong, uncharged Lewis acids are given in WO 00/31090.

Particular preference is given to compounds of the formula (VIII) in which X¹, X² and X³ are identical, preferably tris(pentafluorophenyl)borane.

Further strong uncharged Lewis acids suitable as cation-forming compounds B) are the reaction products from the reaction of a boronic acid with two equivalents of a trialkylaluminum or the reaction products from the reaction of a trialkylaluminum with two equivalents of an acidic fluorinated, in particular perfluorinated, hydrocarbon compound such as pentafluorophenol or bis(pentafluorophenyl)borinic acid.

Suitable ionic compounds having Lewis-acid cations are salt-like compounds of the cation of the formula (IX)

[(Y^{a+})Q₁Q₂... Qz]^{d+} (IX)

where
- Y: is an element of groups 1 to 16 of the Periodic Table of the Elements,
- Q₁ to Q_{z}: are singly negatively charged groups such as C₁-C₂₈-alkyl, C₆-C₁₅-aryl, alkylaryl, arylalkyl, haloalkyl, haloaryl each having from 6 to 20 carbon atoms in the aryl radical and from 1 to 28 carbon atoms in the alkyl radical, C₃-C₁₀₋cycloalkyl which may be substituted by C₁-C₁₀-alkyl groups, halogen, C₁-C₂₈₋alkoxy, C₆-C₁₅-aryloxy, silyl or mercapto groups,
- a: is an integer from 1 to 6 and
- z: is an integer from 0 to 5,
- d: corresponds to the difference a - z, but d is greater than or equal to 1.

Particularly useful cations are carbonium cations, oxonium cations and sulfonium cations and also cationic transition metal complexes. Particular mention may be made of the triphenylmethyl cation, the silver cation and the 1,1'-dimethylferrocenyl cation. They preferably have noncoordinating counterions, in particular boron compounds as are mentioned in WO 91/09882, preferably tetrakis(pentafluorophenyl)borate.

Salts containing noncoordinating anions can also be prepared by combining a boron or aluminum compound, e.g. an aluminum alkyl, with a second compound which can react to link two or more boron or aluminum atoms, e.g. water, and a third compound which reacts with the boron or aluminum compound to form an ionizing ionic compound, e.g. triphenylchloromethane. In addition, a fourth compound which likewise reacts with the boron or aluminum compound, e.g. pentafluorophenol, can be added.

Ionic compounds containing Brönsted acids as cations preferably likewise have noncoordinating counterions. As Brönsted acids, particular preference is given to protonated amine or aniline derivatives. Preferred cations are N,N-dimethylanilinium, N,N-dimethylcyclohexylammonium and N,N-dimethylbenzylammonium and also derivatives of the latter two.

Preferred ionic compounds B) are, in particular, N,N-dimethylanilinium tetrakis(pentafluorophenyl)borate, N,N-dimethylcyclohexylammonium tetrakis(pentafluorophenyl)borate or N,N-dimethylbenzylammonium tetrakis(pentafluorophenyl)borate,

Two or more borate anions can also be joined to one another, as in the dianion [(C₆F₅)₂B-C₆F₄₋B(C₆F₅)₂]²⁻, or the borate anion can be bound via a bridge having a suitable functional group to a support surface.

Further suitable cation-forming compounds B) are listed in WO 00/31090.

The amount of strong, uncharged Lewis acids, ionic compounds having Lewis-acid cations or ionic compounds containing Brönsted acids as cations is preferably from 0.1 to 20 equivalents, preferably from 1 to 10 equivalents, based on the organometallic transition metal compound of the present invention.

Further suitable cation-forming compounds B) are boron-aluminum compounds such as di[bis(pentafluorophenylboroxy)]methylalane. Such boron-aluminum compounds are disclosed, for example, in WO 99/06414.

It is also possible to use mixtures of all the abovementioned cation-forming compounds B). Preferred mixtures comprise aluminoxanes, in particular methylaluminoxane, and an ionic compound, in particular one containing the tetrakis(pentafluorophenyl)borate anion, and/or a strong uncharged Lewis acid, in particular tris(pentafluorophenyl)borane.

Preference is given to using both the novel organometallic transition metal compound and the cation-forming compounds B) in a solvent, with aromatic hydrocarbons having from 6 to 20 carbon atoms, in particular xylene and toluene, being preferred.

The catalyst may further comprise, as additional component C), a metal compound of the formula (X),

M⁵(R²²)ᵣ(R²³)ₛ(R²⁴)ₗ (X)

where
- M⁵: is an alkali metal, an alkaline earth metal or a metal of group 13 of the Periodic Table, i.e. boron, aluminum, gallium, indium or thallium,
- R²²: is hydrogen, C₁-C₁₀-alkyl, C₆-C₁₅-aryl, alkylaryl or arylalkyl each having from 1 to 10 carbon atoms in the alkyl part and from 6 to 20 carbon atoms in the aryl part,
- R²³ and R²⁴: are each hydrogen, halogen, C₁-C₁₀-alkyl, C₆-C₁₅-aryl, alkylaryl, arylalkyl or alkoxy each having from 1 to 10 carbon atoms in the alkyl radical and from 6 to 20 carbon atoms in the aryl radical,
- r: is an integer from 1 to 3

and
- s and t: are integers from 0 to 2, where the sum r+s+t corresponds to the valence of M⁵,

with the component C) not being identical to the component B). It is also possible to use mixtures of various metal compounds of the formula (X).

Among the metal compounds of the formula (X), preference is given to those in which
- M⁵: is lithium, magnesium or aluminum and
- R²³ and R²⁴: are each C₁-C₁₀-alkyl.

Particularly preferred metal compounds of the formula (X) are n-butyllithium, n-butyl-n-octylmagnesium, n-butyl-n-heptylmagnesium, tri-n-hexylaluminum, triisobutylaluminum, triethylaluminum and trimethylaluminum and mixtures thereof.

If a metal compound is used as component C), it is preferably present in the catalyst in such an amount that the molar ratio of M⁵ from formula (X) to transition metal M¹ from the organometallic transition metal compound of the present invention is from 800:1 to 1:1, in particular from 200:1 to 2:1.

Particular preference is given to a catalyst system comprising an organometallic transition metal compound according to the present invention (component A) and at least one cocatalyst (component B) and, in addition, a support (component D).

To obtain such a supported catalyst system, the unsupported catalyst system can be reacted with a support (component D). The order in which component D), the organometallic transition metal compound of the present invention and the cocatalyst are combined is in principle immaterial. The organometallic transition metal compound and the cocatalyst can be fixed to the supports either independently of one another or simultaneously. After the individual process steps, the solid can be washed with suitable inert solvents such as aliphatic or aromatic hydrocarbons.

As component D), preference is given to using finely divided supports which can be any organic or inorganic, inert solids. In particular, the component D) can be a porous support such as talc, a sheet silicate, an inorganic oxide or a finely divided polymer powder (e.g. polyolefin).

Suitable inorganic oxides may be found among oxides of the elements of groups 2, 3, 4, 5, 13, 14, 15 and 16 of the Periodic Table of the Elements. Examples of oxides preferred as supports include silicon dioxide, aluminum oxide and also mixed oxides of the elements calcium, aluminum, silicon, magnesium and titanium and also corresponding oxide mixtures. Other inorganic oxides which can be used alone or in combination with the abovementioned preferred oxidic supports are, for example, MgO, ZrO₂, TiO₂ or B₂O₃. A preferred mixed oxide is, for example, calcined hydrotalcite.

The support materials used preferably have a specific surface area in the range from 10 to 1000 m²/g, a pore volume in the range from 0.1 to 5 mUg and a mean particle size of from 1 to 500 µm. Preference is given to supports having a specific surface area in the range from 50 to 500 m²/g, a pore volume in the range from 0.5 to 3.5 ml/g and a mean particle size in the range from 5 to 350 µm. Particular preference is given to supports having a specific surface area in the range from 200 to 400 m²/g, a pore volume in the range from 0.8 to 3.0 ml/g and a mean particle size of from 10 to 100 µm.

The inorganic support can be subjected to a thermal treatment, e.g. for the removal of adsorbed water. Such a drying treatment is generally carried out at from 80 to 300°C, preferably from 100 to 200°C, with drying at from 100 to 200°C preferably being carried out under reduced pressure and/or a blanket of inert gas (e.g. nitrogen), or the inorganic support can be calcined at from 200 to 1000°C to set, if appropriate, the desired structure of the solid and/or the desired OH concentration on the surface. The support can also be treated chemically using customary desiccants such as metal alkyls, preferably aluminum alkyls, chlorosilanes or SiCl₄, or else methylaluminoxane. Such treatment methods are described, for example, in WO 00/31090.
The inorganic support material can also be modified chemically. For example, the treatment of silica gel with NH₄SiF₆ leads to fluorination of the silica gel surface and the treatment of silica gels with silanes containing nitrogen-, fluorine- or sulfur-containing groups leads to correspondingly modified silica gel surfaces.

Organic support materials such as finely divided polyolefin powders (e.g. polyethylene, polypropylene or polystyrene) can also be used and should preferably likewise be freed of adhering moisture, solvent residues or other impurities by appropriate purification and drying operations before use. It is also possible to use functionalized polymer supports, e.g. ones based on polystyrenes, via whose functional groups, for example ammonium or hydroxy groups, at least one of the catalyst components can be immobilized.

In a preferred method of preparing the supported catalyst system, at least one of the organometallic transition metal compounds of the present invention is brought into contact with at least one cocatalyst component B) in a suitable solvent, preferably giving a soluble reaction product, an adduct or a mixture.

The preparation obtained in this way is then mixed with the dehydrated or passivated support material, the solvent is removed and the resulting supported organometallic transition metal compound catalyst system is dried to ensure that all or most of the solvent is removed from the pores of the support material. The supported catalyst is obtained as a free-flowing powder. Examples of the industrial implementation of the above process are described in WO 96/00243, WO 98/40419 or WO 00/05277.

A further preferred embodiment comprises firstly applying the cation-forming compound to the support component and subsequently bringing this supported cation-forming compound into contact with the organometallic transition metal compound of the present invention.

Thus, useful cocatalyst systems B) likewise include combinations which are obtained by combining the following components:
1. at least one defined boron or aluminum compound,
2. at least one uncharged compound which has at least one acidic hydrogen atom,
3. at least one support, preferably an inorganic oxidic support, and optionally a base, preferably an organic nitrogen-containing base such as an amine, an aniline derivative or a nitrogen heterocycle.

The boron or aluminum compounds used in the preparation of the supported cocatalysts are preferably compounds of the formula XI 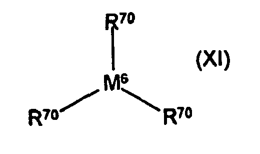
where
- R⁷⁰: are identical or different and are each hydrogen, halogen, C₁-C₂₀-alkyl, C₁-C₂₀-haloalkyl, C₁-C₁₀-alkoxy, C₆-C₂₀-aryl, C₆-C₂₀-haloaryl, C₆-C₂₀-aryloxy, C₇-C₄₀-arylalkyl, C₇-C₄₀₋haloarylalkyl, C₇-C₄₀-alkylaryl, C₇-C₄₀-haloalkylaryl, or R⁷⁰ is an OSiR⁷⁷₃ group, where
- R⁷⁷: are identical or different and are each hydrogen, halogen, C₁-C₂₀-alkyl, C₁-C₂₀-haloalkyl, C₁-C₁₀-alkoxy, C₆-C₂₀-aryl, C₆-C₂₀-haloaryl, C₆-C₂₀-aryloxy, C₇-C₄₀-arylalkyl, C₇-C₄₀₋haloarylalkyl, C₇-C₄₀-alkylaryl, C₇-C₄₀-haloalkylaryl, preferably hydrogen, C₁-C₈-alkyl or C₇-C₂₀-arylalkyl, and
- M⁶: is boron or aluminum, preferably aluminum.

Particularly preferred compounds of the formula XI are trimethylaluminum, triethylaluminum and triisobutylaluminum.

The uncharged compounds which have at least one acidic hydrogen atom and can react with compounds of the formula (XI) are preferably compounds of the formulae XII, XIII or XIV, 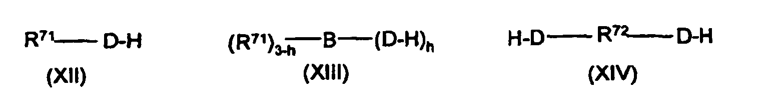
where
- R⁷¹: are identical or different and are each hydrogen, halogen, a boron-free C₁-C₄₀ group such as C₁-C₂₀-alkyl, C₁-C₂₀-haloalkyl, C₁-C₁₀-alkoxy, C₆-C₂₀-aryl, C₆-C₂₀-haloaryl, C₈-C₂₀₋aryloxy, C₇-C₄₀-arylalkyl, C₇₋C₄₀-haloarylalkyl, C₇-C₄₀-alkylaryl, C₇-C₄₀-haloalkylaryl, an Si(R⁷³)₃ group or a CH(SiR⁷³₃)₂ group, where
- R⁷³: is a boron-free C₁-C₄₀ group such as C₁-C₂₀-alkyl, C₁-C₂₀-haloalkyl, C₁-C₁₀-alkoxy, C₆-C₂₀₋aryl, C₆-C₂₀-haloaryl, C₆-C₂₀-aryloxy, C₇₋C₄₀-arylalkyl, C₇-C₄₀-haloarylalkyl, C₇-C₄₀₋alkylaryl, C₇-C₄₀-haloalkylaryl, and
- R⁷²: is a divalent C₁-C₄₀ group such as C₁-C₂₀-alkylene, C₁-C₂₀-haloalkylene, C₆-C₂₀-arylene, C₆-C₂₀-haloarylene, C₇-C₄₀-arylalkylene, C₇-C₄₀-haloarylalkylene, C₇-C₄₀-alkylarylene, C₇₋C₄₀-haloalkylarylene,
- D: is an element of group 16 of the Periodic Table of the Elements or an NR⁷⁴ group, where R⁷⁴ is hydrogen or a C₁-C₂₀-hydrocarbon radical such as C₁-C₂₀-alkyl or C₆-C₂₀-aryl, preferably oxygen, and
- h: is 1 or 2.

Suitable compounds of the formula (XII) are water, alcohols, phenol derivatives, thiophenol derivatives or aniline derivatives, with the halogenated and in particular the perfluorinated alkyls and phenols being of special significance. Examples of particularly useful compounds are pentafluorophenol, 1,1-bis(pentafluorophenyl)methanol and 4-hydroxy-2,2',3,3',4,4',5,5',6,6'-nonafluorobiphenyl.
Suitable compounds of the formula (XIII) are boronic acids and borinic acids, in particular borinic acids having perfluorinated aryl radicals, for example (C₆F₅)₂BOH.
Suitable compounds of the formula (XIV) are dihydroxy compounds in which the divalent carbon-containing group is preferably halogenated, in particular perfluorinated. An example of such a compound is 4,4'-dihydroxy-2,2',3,3',5,5',6,6'-octafluorobiphenyl hydrate.

Examples of combinations of compounds of the formula (XI) with compounds of the formula (XII) or (XIV) are trimethylaluminum/pentafluorophenol, trimethylaluminum/1-bis(pentafluorophenol)methanol, trimethylaluminum/4-hydroxy-2,2',3,3',4,4',5,5',6,6'-nonafluorobiphenyl, triethylaluminum/pentafluorophenol, triisobutylaluminum/pentafluorophenol and triethylaluminum/4,4'-dihydroxy-2,2',3,3',5,5',6,6'-octafluorobiphenyl hydrate, with, for example, reaction products of the following types being able to be formed.

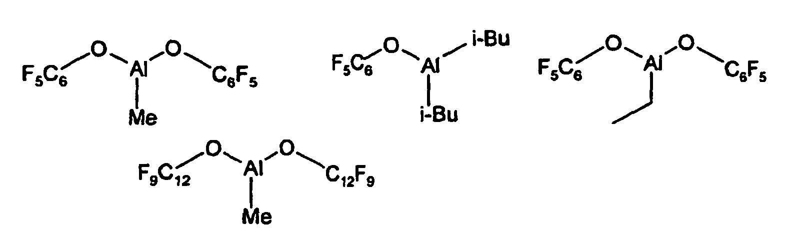

Examples of reaction products from the reaction of at least one compound of the formula (XI) with at least one compound of the formula (XIII) are:

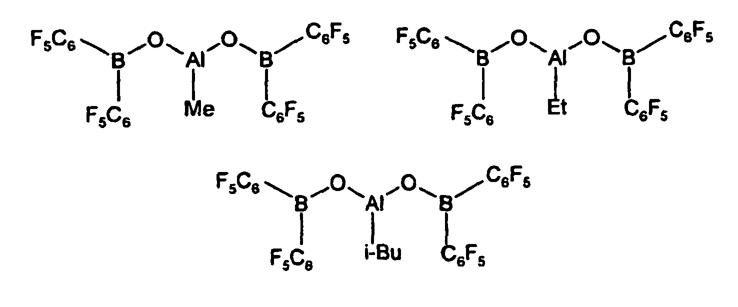

In principle, the components can be combined in any way.

The reaction products from the reaction of at least one compound of the formula XI with at least one compound of the formula XII, XIII or XIV and optionally the organic nitrogen base may additionally be combined with an organometallic compound of the formula VI, VII, VIII and/or X so as then to form, together with the support, the supported cocatalyst system B).

In a preferred embodiment, the components 1 (formula XI) and 2 (formula XII, XIII or XIV) and the components 3 (support) and 4 (base) are combined separately and subsequently reacted with one another, with the reaction preferably taking place in an inert solvent or suspension medium. The supported cocatalyst B) formed can be freed of the inert solvent or suspension medium before it is reacted with the organometallic transition metal component of the present invention and any component C) to form the catalyst system.

It is also possible firstly to prepolymerize the catalyst solid with α-olefins, preferably linear C₂-C₁₀₋1-alkenes and in particular ethylene or propylene, and then to employ the resulting prepolymerized catalyst solid in the actual polymerization. The mass ratio of catalyst solid used in the prepolymerization to polymerized-on monomer is usually in the range from 1:0.1 to 1:200.

Furthermore, a small amount of an olefin, preferably an α-olefin, for example vinylcyclohexane, styrene or phenyldimethylvinylsilane, as modified component, an antistatic or a suitable inert compound such as a wax or oil can be added as additive during or after the preparation of the supported catalyst system. The molar ratio of additives to the organometallic transition metal compound of the present invention is usually from 1:1000 to 1000:1, preferably from 1:5 to 20:1.

### Polymerization process

The present invention also provides a process for preparing polyolefins by polymerization, i.e. homopolymerization or copolymerization, of at least one olefin in the presence of a catalyst system comprising at least one of the novel organometallic transition metal compounds of the formula (I).

In general, the catalyst system is used together with a further metal compound C') of the formula (X), which may be different from the metal compound(s) C) of the formula (X) used in the preparation of the catalyst system, as constituent of a catalyst system for the polymerization or copolymerization of olefins. The further metal compound is generally added to the monomer or the suspension medium and serves to free the monomer of substances which may adversely affect the catalyst activity. It is also possible for one or more further cation-forming compounds B) to be additionally added to the catalyst system during the polymerization process.

The olefins can be functionalized, olefinically unsaturated compounds such as esters or amide derivatives of acrylic or methacrylic acid, for example acrylates, methacrylates or acrylonitrile, or nonpolar olefinic compounds, including aryl-substituted α-olefins.

Preference is given to polymerizing olefins of the formula R^{m}-CH=CH-Rⁿ, where R^{m} and Rⁿ are identical or different and are each hydrogen or a carbon-containing radical having from 1 to 20 carbon atoms, in particular from 1 to 10 carbon atoms, and R^{m} and Rⁿ together with the atoms connecting them may form one or more rings.

Examples of such olefins are 1-olefins having from 2 to 40, preferably from 2 to 10, carbon atoms, e.g. ethylene, propylene, 1-butene, 1-pentene, 1-hexene, 1-heptene, 1-octene, 1-decene or 4-methyl-1-pentene, or unsubstituted or substituted vinylaromatic compounds such as styrene and styrene derivatives, or dienes such as 1,3-butadiene, 1,4-hexadiene, 1,7-octadiene, 5-ethylidene-2-norbomene, norbomadiene, ethylnorbomadiene, or cyclic olefins such as norbomene, tetracyclododecene or methylnorbomene.

The catalyst system of the present invention is particularly preferably used for homopolymerizing propylene or ethylene or copolymerizing ethylene with C₃-C₈-α-olefins such as propylene, 1-butene, 1-pentene, 1-hexene and/or 1-octene and/or cyclic olefins such as norbomene and/or dienes having from 4 to 20 carbon atoms, e.g. 1,4-hexadiene, norbomadiene, ethylidenenorbomene or ethylnorbomadiene or, particularly preferably, for copolymerizing propylene with ethylene and/or 1-butene. Examples of copolymers which can be obtained in this way are propylene-ethylene, propylene-1-butene, ethylene-1-hexene and ethylene-1-octene copolymers and ethylene-propylene-ethylidenenorbomene or ethylene-propylene-1,4-hexadiene terpolymers.

The polymerization can be carried out in a known manner in bulk, in suspension, in the gas phase or in a supercritical medium in the customary reactors used for the polymerization of olefins. It can be carried out batchwise or preferably continuously in one or more stages. Solution processes, suspension processes, stirred gas-phase processes or gas-phase fluidized-bed processes are all possible. As solvents or suspension media, it is possible to use inert hydrocarbons, for example isobutane, or else the monomers themselves.

The polymerizations can be carried out at from -60 to 300°C and pressures in the range from 0.5 to 3000 bar. Preference is given to temperatures in the range from 50 to 200°C, in particular from 60 to 100°C, at pressures in the range from 5 to 100 bar, in particular from 15 to 70 bar. The mean residence times are usually from 0.5 to 5 hours, preferably from 0.5 to 3 hours. Hydrogen can be used in the polymerization as molar mass regulator and/or to increase the activity. Furthermore, use can also be made of customary additives such as antistatics. The catalyst system of the present invention can be used directly for the polymerization, i.e. it is introduced in pure form into the polymerization system, or it is admixed with inert components such as paraffins, oils or waxes to improve its meterability.

The novel organometallic transition metal compounds of the formula (I) or the catalyst systems in which they are present are very particularly useful for preparing propylene-ethylene copolymers or polypropylene/propylene-ethylene copolymer mixtures.

The invention therefore also provides a process for preparing propylene-ethylene copolymers or polypropylenelpropylene-ethylene copolymer mixtures in the presence of a catalyst system as described above.

Ethylene-propylene copolymers having an extraordinarily high stereospecificity and regiospecificity and high contents of copolymerized ethylene are obtained at a comparatively low ethylene partial pressure when using the catalyst systems of the present invention. While conventional metallocenes such as dimethylsilanediylbis(2-methyl-4-phenylindenyl)zirconium dichloride give copolymerized ethylene contents up to 10% by weight, the catalysts of the present invention give contents of 10-20% by weight.

The copolymer obtained using the catalyst systems of the present invention has a high ethylene content combined with a high isotacticity of the polypropylene part.

The polymers prepared by the process of the present invention are suitable for producing hard and stiff shaped bodies having a high ultimate tensile strength, e.g. fibers, filaments, injection-molded parts, films, sheets or large hollow bodies (e.g. pipes). The moldings have, in particular, a high toughness, even at temperatures below 20°C, combined with a high stiffness.

The invention is illustrated by the following examples which do not, however, restrict the scope of the invention.

### Examples

General procedures: Preparation and handling of the organometallic compounds was carried out in the absence of air and moisture under argon (Schlenk technique or glove box). All solvents required were purged with argon and dried over molecular sieves before use. The preparation of substituted 1-indanones was carried out by a method analogous to that of WO 98/40331. The dimethylchlorosilyl-substituted indenes were synthesized as described in DE 19936185 or by methods analogous to the method described there.

### Example 1: 7-(4'-tert-Butylphenyl)-1,2-dimethyl-1-indene

In a 250 ml three-necked flask, 6.1 g (250 mmol) of magnesium turnings were covered with 10 ml of diethyl ether, and 1 ml of methyl iodide was then added. As soon as the reaction started, the remaining 14.6 ml of methyl iodide (total of 250 mmol) in 50 ml of diethyl ether were added at such a rate that the solution boiled gently. After the addition was complete, the mixture was stirred under reflux for another 30 minutes. A solution of 13.9 g (50 mmol) of 7-(4'-tert-butylphenyl)-2-methyl-1-indanone in 60 ml of diethyl ether was then added dropwise at 0°C over a period of 30 minutes. The mixture was stirred for another 1 hour at 0°C and for 15 minutes at room temperature and ice was then carefully added at room temperature. 100 ml of 6N HCl were then added dropwise at room temperature. After the reaction was complete (TLC monitoring), 100 ml of water and 100 ml of diethyl ether were added. The phases were separated and the aqueous phase was extracted three times with 50 ml each time of diethyl ether. The combined organic phases were dried over magnesium sulfate and the solvent was removed under reduced pressure. The red oil (13.6 g) obtained in this way was recrystallized from 75 ml of ethanol. 7-(4'-tert-Butylphenyl)-1,2-dimethyl-1-indene was obtained in a yield of 10.1 g (36.5 mmol/73%) and a purity of 99% (GC). ¹H-NMR (400 MHz, CDCl₃): 7.50-7.20 (m, 7H, aromat. H), 3.41 (s, 2H, benzyl. H), 2.13 (s, 3H, methyl), 1.61 (s, 3H, methyl), 1.49 (s, 9H, tert-butyl)ppm.

### Example 2: Preparation of dimethylsilanediylbis(2,3-dimethyl-4-(4'-tert-butylphenyl)-1-indene)

8.8 ml of n-BuLi (22 mmol, 2.5 M in toluene) were added dropwise at 0°C to 5.52 g (20 mmol) of 7-(4'-tert-butylphenyl)-2,3-dimethyl-1-indene in 40 ml of toluene/5 ml of THF over a period of 20 minutes. The mixture was stirred for another 10 minutes at 0°C and 1 hour at 80°C. After cooling to room temperature, the red solution was added at 0°C to a solution of 1.29 g (10 mmol) of dimethyldichlorosilane in 5 ml of toluene. The solution was stirred for another 10 minutes at 0°C and then for 2 hours at 75°C. The yellow suspension was added to 30 ml of water. The aqueous phase was extracted three times with 30 ml each time of toluene. The combined organic phases were dried over magnesium sulfate and the solvent was removed under reduced pressure. The resulting brown oil (7.4 g) was purified by flash chromatography on silica gel (eluant dichloromethane/heptane 1:9, then 1:4). Dimethylsitanediylbis(2,3-dimethyl-4-(4'-tert-buty)phenyl)-1-indene) was obtained in a yield of 3.17 g (5.2 mmol/52%) and a purity of 95% (GC) in the form of a yellow oil. ¹H-NMR (400 MHz, CDCl₃): 7.51-6.89 (m, 14H, aromat. H), 3.72, 3.68 (2 × s, 2H), 2.19, 2.13 (2 × s, 6H, methyl), 1.59 (s, 6H, methyl), 1.38 (s, 18H, tert-butyl), 0.01, -0.06, -0.19, -0.31, -0.39 (5 × s, 6H, SiMe₂)ppm.

### Example 3: Preparation of dimethylsilanediylbis(2,3-dimethyl-4-(4'-tert-butylphenyl)indenyl)zirconium dichloride

1.16 ml (2.9 mmol, 2.5 M in toluene) of n-BuLi were added dropwise at 0°C to a solution of 850 mg (1.4 mmol) of dimethylsilanediylbis(2,3-dimethyl-4-(4'-tert-butytphenyl)-1-indene) in 5 ml of diethyl ether over a period of 5 minutes. The red solution was stirred at room temperature for 13 hours. 326 mg (1.4 mmol) of zirconium tetrachloride were then added at 0°C. The mixture was stirred for another 2 hours at room temperature. The orange solid which had precipitated was isolated by filtration through a G3 frit, washed twice with 5 ml each time of diethyl ether and 2 × 5 ml of tetrahydrofuran. After drying in an oil pump vacuum, the complex was obtained in a yield of
494 mg (0.64 mmol/46%) and a purity of >95% (NMR) in the form of an orange powder. ¹H-NMR (400 MHz, CDCl₃): rac: 7.81-6.69 (m, 14H, aromat. H), 2.00 (s, 6H, methyl), 1.70 (s, 6H, methyl), 1.36-1.30 (m, 24H, tert-butyl, SiMe₂)ppm.
Meso: 7.81-6.72 (m, 14H, aromat. H), 2.05 (s, 6H, methyl), 1.78 (s, 6H, methyl), 1.36-1.30 (m, 24H, tert-butyl, SiMe₂)ppm.

### Example 4: Preparation of dimethylsilanediyl(2,3-dimethyl-4(4'-tert-butylphenyl)indenyl)(2-methyl-4-(4'-tert-butylphenyl)indene)

38.0 ml of n-BuLi (95 mmol, 2.5 M in toluene) were added dropwise at room temperature to 25.0 g (90.5 mmol) of 7-(4'-tert-butylphenyl)-2,3-dimethyl-1-indene in 250 ml of toluene/25 ml of THF over a period of 20 minutes. The mixture was stirred for another 2 hours at 80°C, during which time the Li salt formed precipitated. After cooling to room temperature, 32.1 g (90.45 mmol) of 2-methyl-4-(4'-tert-butylphenyl)-1-dimethylchlorosilylindene were added. After stirring for 3 hours at 60°C, thin layer chromatography no longer detected any starting material. The solution was added to 250 ml of water. After extraction with 3 × 100 ml of toluene, the combined organic phases were dried over magnesium sulfate. The solvent was removed under reduced pressure. Drying in an oil pump vacuum gave the product as a yellow, vitreous residue in a yield of 53.9 g (quantitative). ¹H-NMR (400 MHz, CDCl₃): 7.54-7.03 (m, 14H, aromat. H), 6.83 (s, 1H, C=C-H), 3.75-3.28, 2.34-1.95, 1.56-1.48 (3 X m, 11 H, CH₃, benzyl. H), 1.38, 1.37 (2 X s, 18H, tert-butyl), -0.21, -0.23, - 0.25, -0.28 (4 X s, 6H, SiMe₂)ppm.

### Example 5: Preparation of dimethylsilanediyl(2,3-dimethyl-4-(4'-tert-butylphenyl)indenyl)(2-methyl-4-(4'-tert-butylphenyl)indenyl)zinrconium dichloride

72.5 ml (181 mmol, 2.5 M in toluene) of n-BuLi were added dropwise at room temperature to a solution of 53.9 g (90.5 mmol) of dimethylsilanediyl(2,3-dimethyl-4-(4'-tert-butylphenyl)indenyl)(2-methyl-4-(4'-tert-butylphenyl)indene) in 540 ml of diethyl ether over a period of 15 minutes. The red solution was stirred at room temperature for 12 hours. 22.2 g (95.1 mmol) of zirconium tetrachloride were then added at 0°C. The mixture was stirred for another 2 hours at room temperature. The orange solid which had precipitated was isolated by filtration through a G3 frit, washed twice with 100 ml each time of diethyl ether and twice with 200 ml each time of tetrahydrofuran. After drying in an oil pump vacuum, the complex was obtained in a yield of 34.8 g (46.1 mmol/51%) and a purity of >95% (NMR) in the form of an orange powder. ¹H-NMR (400 MHz, CDCl₃): pseudo-rac: 7.67-6.99 (m, 14H, aromat. H), 6.97 (s, 1H, C=C-H), 2.25, 1.97, 1.67 (3 X s, 9H, CH₃), 1.34 (s, 9H, tert-butyl), 1.33 (s, 6H, SiMe₂) 1.31 (s, 9H, tert-butyl)ppm. Pseudo-meso: 7.69-6.87 (m, 14H, aromat. H), 6.76 (s, 1H. C=C-H), 2.31, 2.15, 1.74 (3 X s, 9H, CH₃), 1.48 (s, 3H, SiMe), 1.34, 1.31 (2 X s, 18H, tert-butyl), 1.22 (s, 3H, SiMe) ppm.

### Example 6: Preparation of dimethylsilanediyl(2,3-dimethyl-4-(4'-tert-butylphenyl)indenyl)(2-isopropyl-4-(4'-tert-butylphenyl)indene)

7.6 ml of n-BuLi (19.0 mmol, 2.5 M in toluene) were added dropwise at room temperature to 5.0 g (18.1 mmol) of 7-(4'-tert-butylphenyl)-2,3-dimethyl-1-indene in 50 ml of toluene/5 ml of THF over a period of 20 minutes. The mixture was stirred for another 2 hours at 80°C, during which time the Li salt formed precipitated. After cooling to room temperature, 6.93 g (18.1 mmol) of 2-isopropyl-4-(4'-tert-butylphenyl)-1-dimethylchtorosilylindene were added. After stirring for 3 hours at 60°C, thin layer chromatography no longer detected any starting material. The solution was added to 50 ml of water. After extraction with 3 × 50 ml of toluene, the combined organic phases were dried over magnesium sulfate. The solvent was removed under reduced pressure. Drying in an oil pump vacuum gave the product as a yellow, vitreous residue in a yield of 11.67 g (quantitative). ¹H-NMR (400 MHz, CDCl₃): 7.49-7.07 (m, 14H, aromat. H), 6.82 (s, 1H, C=C-H), 3.91-1.45 (m, 9H, CH₃, benzyl. H, isopropyl-H), 1.37, 1.36 (2 X s, 18H, tert-butyl), 1.27-1.05 (m, 6H, isopropyl-CH₃), - 0.19, -0.25, -0.27, -0.32 (4 X s, 6H, SiMe₂) ppm.

### Example 7: Preparation of dimethylsitanediyl(2,3-dimethyl-4-(4'-tert-butylphenyl)indenyl)(2-isopropyl-4-(4'-tert-butylphenyl)indenyl)zirconium dichloride

14.9 ml (37.3 mmol, 2.5 M in toluene) of n-BuLi were added dropwise at room temperature to a solution of 11.6 g (18.7 mmol) of dimethylsilanediyl(2,3-dimethyl-4-(4'-tert-butylphenyl)indenyl)(2-isopropyl-4-(4'-tert-butylphenyl)indene) in 120 ml of diethyl ether over a period of 15 minutes. The red solution was stirred at room temperature for 12 hours. 4.6 g (19.6 mmol) of zirconium tetrachloride were then added at 0°C. The mixture was stirred for another 2 hours at room temperature. The orange solid which had precipitated was isolated by filtration through a G3 frit, washed twice with 30 ml each time of diethyl ether and once with 50 ml of tetrahydrofuran. After drying in an oil pump vacuum, the complex was obtained in a yield of 5.6 g (7.2 mmol/38%) and a purity of >95% (NMR) in the form of an orange powder. ¹H-NMR (400 MHz, CDCl₃): pseudo-rac: 7.70-6.84 (m, 14H, aromat. H), 6.78 (s, 1H, C=C-H), 3.04 (q, 1H, isopropyl-CH), 1.99, 1.69 (2 X s, 6H, CH₃), 1.34 (s, 9H, tert-butyl), 1.32 (s, 6H, SiMe₂) 1.31 (s, 9H, tert-butyl), 1.01 (d, 6H, isopropyl-CH₃) ppm. Pseudo-meso: 7.69-6.88 (m, 14H, aromat. H), 6.75 (s, 1H, C=C-H), 3.33 (q, 1H, isopropyl-CH), 2.07, 1.75 (2 X s, 6H, CH₃), 1.51 (s, 3H, SiMe), 1.35, 1.30 (2 X s, 18H, tert-butyl), 1.24 (s, 3H, SiMe), 1.15 (d, 6H, isopropyl-CH₃) ppm.

### Example 8: 7-(4'-tert-butylphenyl)-1-methyl-2-isopropyl-1-indene

In a 250 ml three-necked flask, 6.0 g (245 mmol) of magnesium turnings were covered with 10 ml of diethyl ether, and 1 ml of methyl iodide was then added. As soon as the reaction started, the remaining 14.2 ml of methyl iodide (total of 245 mmol) in 50 ml of diethyl ether were added at such a rate that the solution boiled gently. After the addition was complete, the mixture was stirred under reflux for another 30 minutes. A solution of 13.9 g (49 mmol) of 7-(4'-tert-butylphenyl)-2-isopropyl-1-indanone in 60 ml of diethyl ether was then added dropwise at 0°C over a period of 30 minutes. The mixture was stirred for another 1 hour at 0°C and 15 minutes at room temperature and ice was then added carefully at room temperature. 108 ml of 6N HCl were then added dropwise at room temperature. After the reaction was complete (TLC monitoring), 100 ml of water and 100 ml of diethyl ether were added. The phases were separated and the aqueous phase was extracted three times with 50 ml each time of diethyl ether. The combined organic phases were dried over magnesium sulfate and the solvent was removed under reduced pressure. The resulting red oil (quantitative) was recrystallized from 75 ml of ethanol. 7-(4'-tert-Butylphenyl)-1-methyl-2-isopropyl-1-indene was obtained in a yield of 7.6 g (25 mmol/51%) and a purity of 96% (GC). ¹H-NMR (400 MHz, CDCl₃): 7.45-7.08 (m, 7H, aromat. H), 3.31 (s, 2H, benzyl. H), 2.99 (q, 1H, isopropyl-C-H), 1.51 (s, 3H, methyl), 1.37 (s, 9H, tert-butyl), 1.12 (d, 6H, isopropyl-CH₃) ppm.

### Example 9: Preparation of dimethylsilanediyl(2-isopropyl-3-methyl-4-(4'-tert-butylphenyl)indenyl)(2-methyl-4-(4'-tert-butylphenyl)indene)

10.5 ml of n-BuLi (26.3 mmol, 2.5 M in toluene) were added dropwise at room temperature to 7.6 g (25.0 mmol) of 7-(4'-tert-butylphenyl)-1-methyl-2-isopropyl-1-indene in 80 ml of toluene/8 ml of THF over a period of 20 minutes. The mixture was stirred for another 2 hours at 80°C, during which time the Li salt formed precipitated. After cooling to room temperature, 8.9 g (25.0 mmol) of 2-methyl-4-(4'-tert-butylphenyl)-1-dimethylchlorosilylindene were added. After stirring for 3 hours at 60°C. thin layer chromatography no longer detected any starting material. The solution was added to 50 ml of water. After extraction with 3 × 50 ml of toluene, the combined organic phases were dried over magnesium sulfate. The solvent was removed under reduced pressure. After drying in an oil pump vacuum, the product was obtained as a yellow, vitreous residue in a yield of 15.6 g (quantitative). ¹H-NMR (400 MHz, CDCl₃): 7.51-7.09 (m, 14H, aromat. H), 6.76 (s, 1H, C=C-H), 3.88-1.47 (m, 9H, CH₃, benzyl, H, isopropyl-H), 1.38, 1.35 (2 X s, 18H, tert-butyl), 1.29-1.04 (m, 6H, isopropyl-CH₃), -0.20, -0.25, -0.28, -0.33 (4 X s, 6H, SiMe₂) ppm.

### Example 10: Preparation of dimethylsilanediyl(2-isopropyl-3-methyl-4-(4'-tert-butylphenyl)indenyl)(2-methyl-4-(4'-tert-butylphenyl)indenyl)zirconium dichloride

10.5 ml (26.3 mmol, 2.5 M in toluene) of n-BuLi were added dropwise at room temperature to a solution of 15.6 g (25.0 mmol) of dimethylsilanediyl(2-isopropyl-3-methyl-4-(4'-tert-butylphenyl)indenyl)(2-methyl-4-(4'-tert-butylphenyl)indene) in 125 ml of diethyl ether over a period of 15 minutes. The red solution was stirred for 12 hours at room temperature. 5.8 g (25.0 mmol) of zirconium tetrachloride were then added at 0°C. The mixture was stirred for another 2 hours at room temperature. The orange solid which had precipitated was isolated by filtration through a G3 frit, washed twice with 30 ml each time of diethyl ether and once with 50 ml of tetrahydrofuran. After drying in an oil pump vacuum, the complex was obtained in a yield of 9.2 g (11.7 mmol/47%) and a purity of >95% (NMR) in the form of an orange powder. ¹H-NMR (400 MHz, CDCl₃): pseudo-rac: 7.73-6.81 (m, 14H, aromat H), 6.79 (s, 1H. C=C-H), 3.07 (q, 1H, isopropyl-CH), 2.01, 1.71 (2 X s, 6H, CH₃), 1.35 (s, 9H, tert-butyl), 1.33 (s, 6H, SiMe₂) 1.32 (s, 9H, tert-butyl), 0.99 (d, 6H, isopropyl-CH₃) ppm. Pseudo-meso: 7.73-6.84 (m, 14H, aromat. H), 6.74 (s, 1H, C=C-H), 3.35 (q, 1H, isopropyl-CH), 2.09, 1.77 (2 X s, 6H, CH₃), 1.53 (s, 3H, SiMe), 1.35, 1.31 (2 X s, 18H, tert-butyl), 1.26 (s. 3H, SiMe), 1.16 (d, 6H, isopropyl-CH₃) ppm.

### Polymerization

Abbreviations used:
- pp =: polypropylene
- MC =: metallocene
- cat =: supported catalyst system
- h =: hour
- standard dm³ =: standard liters
- rpm =: revolutions per minute
- VN =: viscosity number in cm³/g
- M_{w} =: weight average molar mass in g/mol
- M_{w}/Mₙ =: molar mass distribution, determined by gel permeation chromatography
- BD =: bulk density in g/dm³
- m.p. =: melting point in °C, determined by differential scanning calorimetry (DSC) at a heating and cooling rate of 10°C/min.
- TT: triad tacticity in percent determined by ¹³C-NMR spectroscopy
- RI =: reverse insertions in %, determined by ¹³C-NMR spectroscopy

### Example 11:

### Preparation of the supported catalyst system:

75.5 mg (0.10 mmol) of dimethylsilanediyl(2,3-dimethyl-4-(4'-tert-butylphenyl)indenyl)(2-methyl-4-(4'-tert-butylphenyl)indenyl)zirconium dichloride were dissolved at room temperature in 4.3 cm³ (20 mmol of Al) of 30% strength methylaluminoxane solution in toluene (Albemarle Corporation, Baton Rouge, Louisiana, USA). The solution was diluted with 3.7 cm³ of toluene and stirred for 1 hour at 25°C while being protected from light. This solution was added a little at a time while stirring to 4.0 g of SiO₂ (grade MS 948, W.R. Grace, Davison Chemical Division, Baltimore, Maryland, USA, pore volume = 1.6 ml/g, calcined at 600°C) and the mixture was stirred for another 10 minutes after the addition was complete. The ratio of volume of solution to total pore volume of the support material was 1.25. The mixture was subsequently dried for 4 hours at 40°C and 10⁻³ mbar. 5.5 g of a free-flowing powder were obtained.

### Polymerization:

A dry 16 dm³ reactor which had been flushed firstly with nitrogen and subsequently with propylene was charged with 10 dm³ of liquid propylene. 8 cm³ of 20% strength triethylaluminum solution in Varsol (Witco) were added as scavenger and the mixture was stirred at 30°C for 15 minutes. A suspension of 2 g of the supported metallocene catalyst in 20 cm³ of Exxsol was subsequently introduced into the reactor, the reaction mixture was heated to the polymerization temperature of 70°C and the polymerization system was held at 70°C for 1 hour. The polymerization was stopped by venting and the polymer obtained was dried under reduced pressure. This gave 2.9 kg of polypropylene powder.
The catalyst activity was 105 kg of PP/(g of MC × h) or 1.5 kg of PP/(g of cat × h): The isotactic polypropylene prepared had the following properties: m.p. = 148°C. M_{w} = 2.6×10⁵ g/mol, M_{w}/Mₙ = 5.0.

### Example 12:

### Preparation of the supported catalyst system:

78.3 mg (0.10 mmol) of dimethylsilanediyl(2,3-dimethyl-4-(4'-tert-butylphenyl)indenyl)(2-isopropyl-4-(4'-tert-butylphenyl)indenyl)zirconium dichloride (example 10) were dissolved at room temperature in 4.3 cm³ (20 mmol of Al) of 30% strength methylaluminoxane solution in toluene (Albemarle Corporation, Baton Rouge, Louisiana, USA). The solution was diluted with 3.7 cm³ of toluene and stirred for 1 hour at 25°C while being protected from light This solution was added a little at a time while stirring to 4.0 g of SiO₂ (grade MS 948, W.R. Grace, Davison Chemical Division, Baltimore, Maryland, USA, pore volume = 1.6 ml/g, calcined at 600°C) and the mixture was stirred for another 10 minutes after the addition was complete. The ratio of volume of solution to total pore volume of the support material was 1.25. The mixture was subsequently dried for 4 hours at 40°C and 10⁻³ mbar. 5.5 g of a free-flowing powder were obtained.

### Polymerization:

A dry 16 dm³ reactor which had been flushed firstly with nitrogen and subsequently with propylene was charged with 10 dm³ of liquid propylene. 8 cm³ of 20% strength triethylaluminum solution in Varsol (Witco) were added as scavenger and the mixture was stirred at 30°C for 15 minutes. A suspension of 2 g of the supported metallocene catalyst in 20 cm³ of Exxsol was subsequently introduced into the reactor, the reaction mixture was heated to the polymerization temperature of 70°C and the polymerization system was held at 70°C for 1 hour. The polymerization was stopped by venting and the polymer obtained was dried under reduced pressure. This gave 2.4 kg of polypropylene powder.
The catalyst activity was 77 kg of PP/(g of MC × h) or 1.2 kg of PP/(g of cat × h).
The isotactic polypropylene prepared had the following properties: m.p. =144°C,M_{w} = 3.7×10⁵ g/mol, M_{w}/Mₙ = 7.

### Example 13:

### Preparation of the supported catalyst system:

78.3 mg (0.10 mmol) of dimethylsilanediyl(2-isopropyl-3-methyl-4-(4'-tert-butytphenyl)indenyl)(2-methyl-4-(4'-tert-butylphenyl)indenyl)zirconium dichloride (example 13) were dissolved at room temperature in 4.3 cm³ (20 mmol of Al) of 30% strength methylaluminoxane solution in toluene (Albemarle Corporation, Baton Rouge, Louisiana, USA). The solution was diluted with 3.7 cm³ of toluene and stirred for 1 hour at 25°C while being protected from light. This solution was added a little at a time while stirring to 4.0 g of SiO₂ (grade MS 948, W.R. Grace, Davison Chemical Division, Baltimore, Maryland, USA, pore volume = 1.6 ml/g, calcined at 600°C) and the mixture was stirred for another 10 minutes after the addition was complete. The ratio of volume of solution to total pore volume of the support material was 1.25. The mixture was subsequently dried for 4 hours at 40°C and 10⁻³ mbar. 5.5 g of a free-flowing powder were obtained.

### Polymerization:

A dry 16 dm³ reactor which had been flushed firstly with nitrogen and subsequently with propylene was charged with 10 dm³ of liquid propylene. 8 cm³ of 20% strength triethylaluminum solution in Varsol (Witco) were added as scavenger and the mixture was stirred at 30°C for 15 minutes. A suspension of 2 g of the supported metallocene catalyst in 20 cm³ of Exxsol was subsequently introduced into the reactor, the reaction mixture was heated to the polymerization temperature of 70°C and the polymerization system was held at 70°C for 1 hour. The polymerization was stopped by venting and the polymer obtained was dried under reduced pressure. This gave 3.4 kg of polypropylene powder.
The catalyst activity was 108 kg of PP/(g of MC × h) or 1.7 kg of PP/(g of cat × h). The isotactic polypropylene prepared had the following properties: m.p. = 146°C, M_{w} = 4.0×10⁵ g/mol, M_{w}/Mₙ = 8.

### Example 14:

### Copolymerization using the catalyst system of Example 11:

A dry 5 dm³ reactor which had been flushed firstly with nitrogen and subsequently with propylene was charged with 3 dm³ of liquid propylene 8 cm³ of 20% strength triethylaluminum solution in Varsol (Witco) were added as scavenger, the reactor was subsequently pressurized with 10 bar of ethylene and the mixture was stirred at 30°C for 15 minutes. A suspension of 1 g of the supported metallocene catalyst in 20 cm³ of Exxsol was subsequently introduced into the reactor, the reaction mixture was heated to the polymerization temperature of 70°C and the polymerization system was held at 70°C for 1 hour. The polymerization was stopped by venting and the polymer obtained was dried under reduced pressure. This gave 2.1 kg of ethylene-propylene copolymer powder.
The catalyst activity was 111 kg of PP-PE copolymer/(g of MC × h) or 2.1 kg of PP-PE/(g of cat × h). The copolymer had the following properties: M_{w} = 0.57 × 10⁵ g/mol, M_{w}/Mₙ = 3.6, M_{w} (copo)/M_{w} (homo) = 0.48, C2 content: 16.7% by weight.

### Comparative example:

A catalyst system was prepared using dimethylsilanediylbis(2-methyl-4-phenylindenyl)zirconium dichloride in a procedure analogous to example 11 and a copolymerization was carried out using a method analogous to example 14. This gave 1.9 kg of ethylene-propylene copolymer powder. The catalyst activity was 130 kg of PP-PE copolymer/(g of MC × h) or 1.9 kg of PP-PE/(g of cat × h). The copolymer had the following properties: M_{w} = 1.39 × 10⁵ g/mol, M_{w}/Mₙ = 2.7, M_{w} (copo)/M_{w} (homo) = 0.26, C2 content: 6.5% by weight

## Claims

1. A transition metal compound of the formula (1) 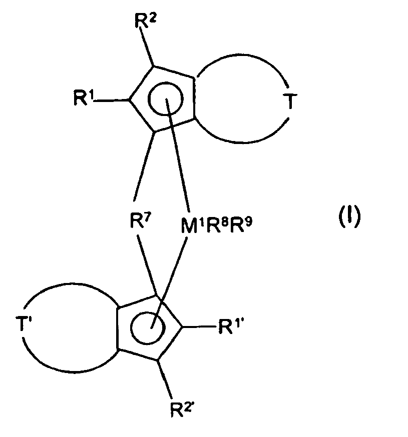
where 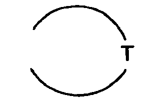
is 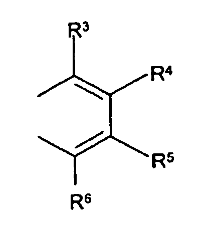
and 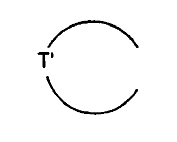
is 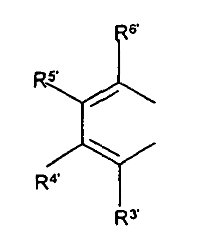
and
M¹ is titanium, zirconium or hafnium;
R¹, R² are identical or different and are each a C₁-C₂₀ group;
R^{1'},R^{2'} are identical or different, identical to or different from R¹ or R² and are each hydrogen or a C₁-C₂₀ group;
R³ is a C₆-C₁₈-aryl group or C₄-C₁₈-heteroaryl; or a fluorinated C₆-C₂₀-aryl or C₇-C₂₀₋alkylaryl, where the aryl part of these groups may bear one or more linear or branched C₁-C₁₈-alkyl, C₁-C₁₈-alkoxy, C₂-C₁₀-alkenyl or C₃-C₁₅-alkylalkenyl groups as substituents, or R³ together with R⁴ forms a monocyclic or polycyclic ring system which may in turn be substituted;
R^{3'} is hydrogen or a C₁-C₄₀ group or R^{3'} together with R^{4'} forms a monocyclic or polycyclic ring system which may in turn be substituted;
R⁴,R^{4'} are identical or different and are each hydrogen or a C₁-C₂₀ group;
R⁵,R^{5'},R⁶,R^{6'} are identical or different and are each hydrogen or a C₁-C₂₀ group;
R⁷ is a bridging structural element between the two indenyl radicals and is selected from the M²R¹⁰R¹¹ group, where M² is silicon, germanium, tin or carbon and R¹⁰ and R¹¹ may be identical or different and are each hydrogen or a C₁-C₂₀₋hydrocarbon-containing group;
R⁸,R⁹ may be identical or different and are each halogen, linear or branched C₁-C₂₀₋alkyl, substituted or unsubstituted phenoxide, or R⁸ and R⁹ are joined to one another and form a monocyclic or polycyclic ring system which may in turn be substituted.

2. A transition metal compound as claimed in claim 1, wherein
M¹ is zirconium;
R¹,R² are identical or different and are each a C₁-C₁₂-alkyl group;
R^{1'},R^{2'} are identical or different and are each hydrogen, methyl, ethyl, n-propyl, isopropyl, n-butyl, isobutyl, tert-butyl, cyclopentyl or cyclohexyl;
R³,R^{3'} are identical or different and are each a C₆-C₁₈-aryl group or two radicals R³ together with R⁴ and/or R^{3'} together with R^{4'} may form a monocyclic or polycyclic ring system which may in turn be substituted, and R^{3'} may also be hydrogen;
R⁴,R^{4'} are identical or different and are either hydrogen or R⁴ together with R³ and/or R^{4'} together with R^{3'} form a monocyclic or polycyclic ring system;
R⁵,R^{5'},R⁶,R^{6'} are identical or different and are each hydrogen, linear or branched C₁-C₁₈₋alkyl, C₂-C₁₀-alkenyl or C₃-C₁₅-alkylalkenyl; C₆-C₂₀-aryl, C₄-C₁₈-heteroaryl, C₇-C₂₀₋arylalkyl; or fluorinated C₁-C₁₂-alkyl, C₂-C₁₀-alkenyl, C₆-C₂₀-aryl or C₇-C₂₀-arylalkyl;
R⁷ is a bridging structural element SiR¹⁰R¹¹ and R¹⁰ and R¹¹ are identical or different and are each a C₁-C₂₀-hydrocarbon-containing group and
R⁸,R⁹ are each chlorine or methyl.

3. A ligand system of the formula (II) or its double bond isomers, 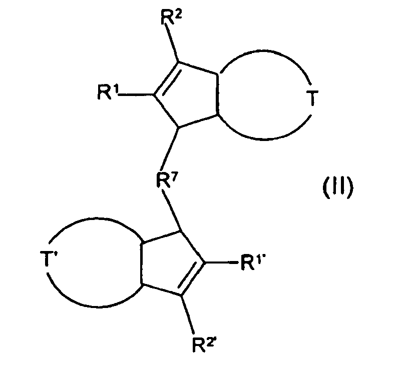
where the variables are as defined for formula (I) of claim 1.

4. A process for preparing ansa-metallocenes of the formula (I) as claimed in claim 1, which comprises the following steps:
a) reaction of a 1-indanone of the formula (III) or (III') with an organometallic compound M³R²ₘHalₙ or M³R^{2'}ₘHalₙ and subsequent elimination to form the substituted indene of the formula (IV) or (IV'), 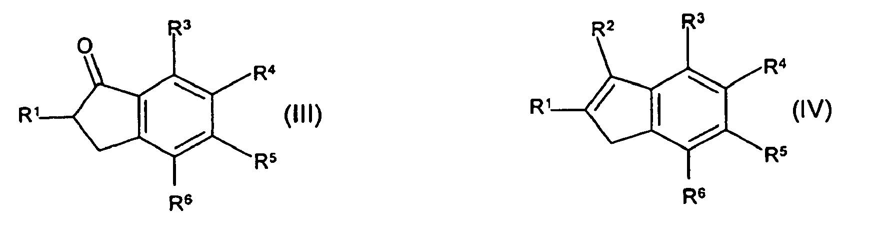 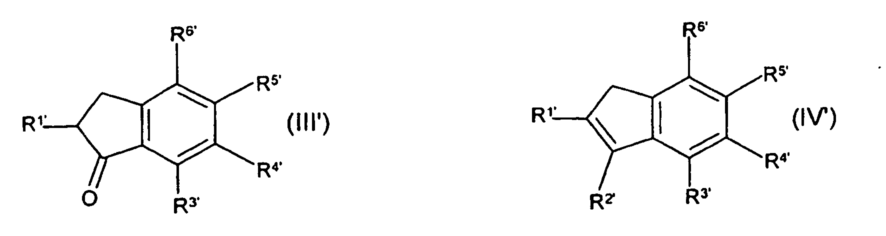
where the variables R¹, R^{1'}, R², R^{2'}, R³, R^{3'}, R⁴, R^{4'}, R⁵, R^{5'}, R⁶ and R^{6'} are as defined for formula (I) of claim 1, M³ is an alkali metal, an alkaline earth metal, aluminum or titanium, Hal is halogen, m is an integer and is equal to or greater than 1 and the sum of m+n corresponds to the valence of M³;
b) deprotonation of the substituted indene of the formula (IV) or (IV') and subsequent reaction of the deprotonated indene with compounds of the type R⁷X₂ to form compounds of the formula (V) or (V') or their double bond isomers, 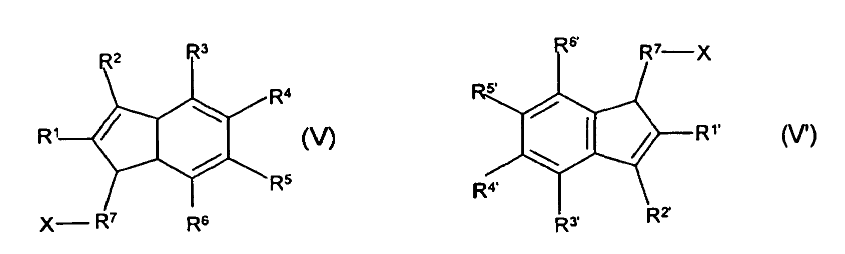 where X is Cl, Br, I or O-tosyl and R⁷ is as defined for formula (I) of claim 1;
c) reaction of the compound of the formula (V) or (V') with a further deprotonated indene which has been obtained by deprotonation of (IV) or (IV') to form the ligand system of the formula (IIa) or its double bond isomers, 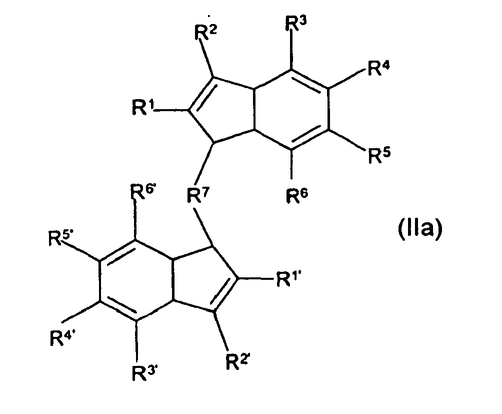
d) deprotonation of the ligand system of the formula (IIa) or its double bond isomers and reaction with compounds of the type X₂M¹R⁸R⁹ to give the ansa-metallocene of the formula (1), where X is as defined for formula (V) and M¹, R⁸ and R⁹ are as defined for formula (I) of claim 1.

5. An indene of the formula (IV) or its double bond isomer, 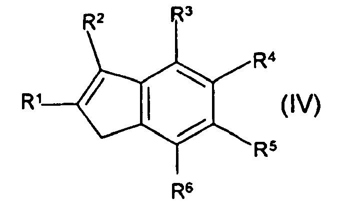
where
R¹, R² are identical or different and are each a C₁-C₂₀ group;
R³ is a C₆-C₁₈-aryl group or C₄-C₁₈-heteroaryl; or a fluorinated C₆-C₂₀-aryl or C₇-C₂₀₋alkylaryl, where the aryl part of these groups may bear one or more linear or branched C₁-C₁₈-alkyl, C₁-C₁₈-alkoxy, C₂-C₁₀-alkenyl or C₃-C₁₅-alkylalkenyl groups as substituents;
R⁴ is hydrogen or a C₁-C₂₀ group;
R⁵, R⁶ are identical or different and are each hydrogen or a C₁-C₂₀ group.

6. A catalyst system comprising one or more compounds of the formula (I) as claimed in claim 1 or 2 and one or more cocatalysts and/or supports.

7. The use of a catalyst system as claimed in claim 6 for the preparation of a polyolefin, in particular a copolymer of various olefins.

8. The use of a compound of the formula (I) as claimed in claim 1 or 2 for the preparation of a polyolefin, in particular a copolymer of various olefins.

9. The use as claimed in claim 7 or 8 for the preparation of ethylene-propylene copolymers.

10. A process for preparing a polyolefin by polymerization of one or more olefins in the presence of one or more compounds of the formula (I) as claimed in claim 1 or 2.

## Patentansprüche

1. Übergangsmetallverbindung der Formel (I) 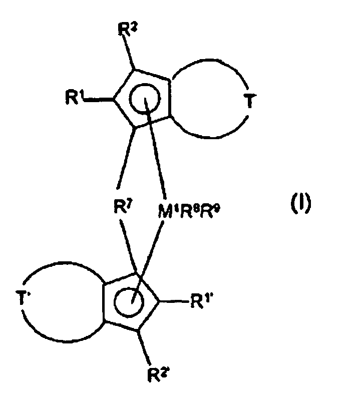
worin 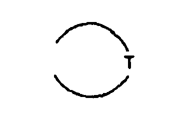 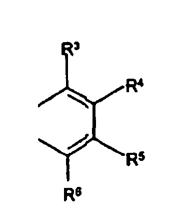
ist und 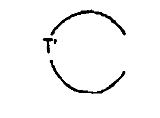 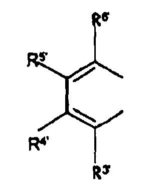
ist und
M¹ Titan, Zirconium oder Hafnium ist;
R¹, R² identisch oder unterschiedlich sind und jeweils eine C₁-C₂₀-Gruppe sind;
R^{1'}, R^{2'} identisch oder unterschiedlich sind, identisch zu oder verschieden von R¹ oder R² sind und jeweils Wasserstoff oder eine C₁-C₂₀-Gruppe sind;
R³ eine C₆-C₁₈-Arylgruppe oder C₄-C₁₈-Heteroarylgruppe ist; oder ein fluoriertes C₆-C₂₀-Aryl- oder C₇-C₂₀-Alkylaryl, wobei der Arylteil dieser Gruppen eine oder mehrere lineare oder verzweigte C₁-C₁₈-Alkyl-, C₁-C₁₈-Alkoxy-, C₂-C₁₀₋Alkenyl- oder C₃-C₁₅-Alkylalkenylgruppen als Substituenten tragen kann, oder R³ zusammen mit R⁴ein monocyclisches oder polycyclisches Ringsystem bildet, welches seinerseits substituiert sein kann;
R^{3'} Wasserstoff oder eine C₁-C₄₀-Gruppe ist oder R^{3'} zusammen mit R^{4'} ein monocyclisches oder polycyclisches Ringsystem bildet, welches seinerseits substituiert sein kann;
R⁴, R^{4'} identisch oder unterschiedlich sind und jeweils Wasserstoff oder eine C₁-C₂₀-Gruppe sind;
R⁵, R^{5'}, R⁶, R^{6'} identisch oder unterschiedlich sind und jeweils Wasserstoff oder eine C₁-C₂₀-Gruppe sind;
R⁷ ein Brückenstrukturelement zwischen den zwei Indenylresten ist und aus der M²R¹⁰R¹¹-Gruppe gewählt ist, wobei M² Silicium, Germanium, Zinn oder Kohlenstoff ist und R¹⁰ und R¹¹ identisch oder unterschiedlich sein können und jeweils Wasserstoff oder eine kohlenwasserstoffhaltige C₁-C₂₀-Gruppe sind;
R⁸, R⁹ identisch oder unterschiedlich sind und jeweils Halogen, lineares oder verzweigtes C₁₋C₂₀-Alkyl, substituiertes oder nicht substituiertes Phenoxid sind oder R⁸ und R⁹ aneinander gebunden sind und ein monocyclisches oder polycyclisches Ringsystem bilden, welches seinerseits substituiert sein kann.

2. Übergangsmetallverbindung, wie in Anspruch 1 beansprucht, wobei
M¹ Zirconium ist;
R¹, R² identisch oder unterschiedlich sind und jeweils eine C₁-C₁₂-Alkylgruppe sind;
R^{1'}, R^{2'} identisch oder unterschiedlich sind und jeweils Wasserstoff, Methyl, Ethyl, n-Propyl, Isopropyl, n-Butyl, Isobutyl, tert-Butyl, Cyclopentyl oder Cyclohexyl sind;
R³, R^{3'} identisch oder unterschiedlich sind und jeweils eine C₆-C₁₈-Arylgruppe sind oder zwei Reste R³ zusammen mit R⁴ und/oder R^{3'} zusammen mit R^{4'} ein monocyclisches oder polycyclisches Ringsystem bilden können, welches seinerseits substituiert sein kann, und R³' ebenfalls Wasserstoff sein kann;
R⁴, R^{4'} identisch oder unterschiedlich sind und entweder Wasserstoff sind oder R⁴ zusammen mit R³ und/oder R^{4'} zusammen mit R^{3'} ein monocyclisches oder polycyclisches Ringsystem bilden;
R⁵, R^{5'}, R⁶, R^{6'} identisch oder unterschiedlich sind und jeweils Wasserstoff, lineares oder verzweigtes C₁-C₁₈-Alkyl, C₂-C₁₀-Alkenyl oder C₃₋C₁₅-Alkylalkenyl; C₆-C₂₀-Aryl, C₄-C₁₈-Heteroaryl, C₇-C₂₀-Arylalkyl; oder fluoriertes C₁-C₁₂-Alkyl, C₂-C₁₀-Alkenyl, C₆-C₂₀-Aryl oder C₇-C₂₀-Arylalkyl sind;
R⁷ ein Brückenstrukturelement SiR¹⁰R¹¹ ist und R¹⁰ und R¹¹ identisch oder unterschiedlich sind und jeweils eine C₁-C₂₀-kohlenwasserstoffhaltige Gruppe sind und
R⁸, R⁹ jeweils Chlor oder Methyl sind.

3. Ligandensystem der Formel (II) oder seine Doppelbindungsisomere 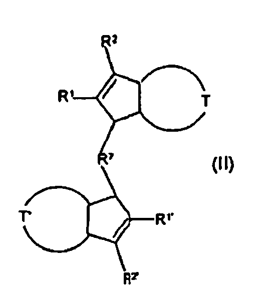
wobei die Variablen wie für die Formel (I) von Anspruch 1 definiert sind.

4. Verfahren zur Herstellung von ansa-Metallocenen der Formel (I), wie in Anspruch 1 beansprucht, welches die folgenden Schritte umfasst:
a) die Reaktion eines 1-Indanons der Formel (III) oder (III') mit einer organometallischen Verbindung M³R²ₘHalₙ oder M³R^{2'}ₘHalₙ und anschließende Eliminierung unter Bildung des substituierten Indens der Formel (IV) oder (IV') 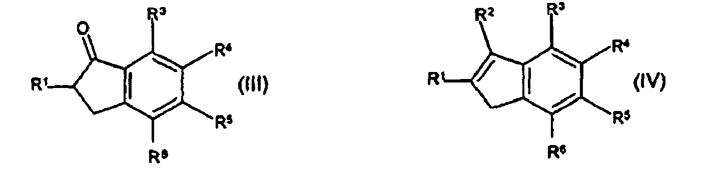 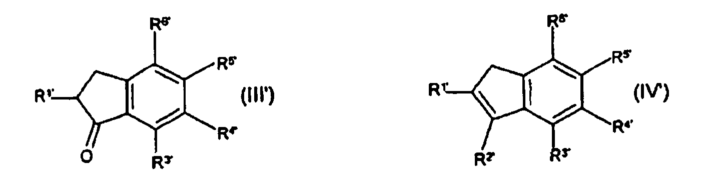 wobei die Variablen R¹, R^{1'}, R², R^{2'}, R³, R^{3'}, R⁴, R^{4'}, R⁵, R^{5'}, R⁶ und R^{6'} wie für die Formel (I) von Anspruch 1 definiert sind, M³ ein Alkalimetall, ein Erdalkalimetall, Aluminium oder Titan ist, Hal Halogen ist, m eine ganze Zahl ist und gleich oder größer als 1 ist, und die Summe von m + n der Valenz von M³ entspricht;
b) die Deprotonierung des substituierten Indens der Formel (IV) oder (IV') und die anschließende Reaktion des deprotonierten Indens mit Verbindungen des Typs R⁷X₂ zur Bildung von Verbindungen der Formel (V) oder (V') oder ihren Doppelbindungsisomeren 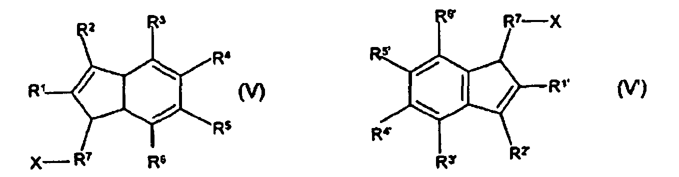 wobei X Cl, Br, I oder O-Tosyl ist und R⁷ wie für die Formel (I) von Anspruch 1 definiert ist;
c) die Umsetzung der Verbindung der Formel (V) oder (V') mit einem weiteren deprotonierten Inden, welches durch Deprotonieren von (IV) oder (IV') erhalten worden ist, zur Bildung des Ligandensystems der Formel (IIa) oder seiner Doppelbindungsisomeren 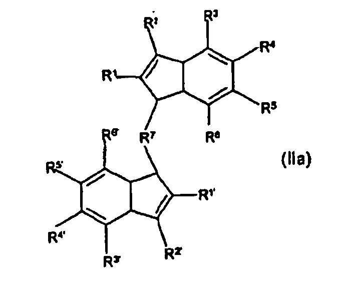
d) die Deprotonierung des Ligandensystems der Formel (IIa) oder seiner Doppelbindungsisomere und die Reaktion mit Verbindungen des Typs X₂M¹R⁸R⁹, um das ansa-Metallocen der Formel (I) zu erhalten, wobei X wie für die Formel (V) definiert ist und M¹, R⁸ und R⁹ wie die Formel (I) von Anspruch 1 definiert sind.

5. Inden der Formel (IV) oder seines Doppelbindungsisomers 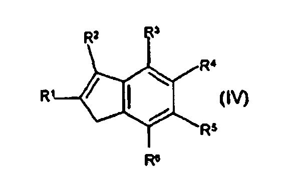
wobei
R¹, R² identisch oder verschieden sind und jeweils eine C₁-C₂₀-Gruppe sind;
R³ eine C₆-C₁₈-Arylgruppe oder C₄-C₁₆-Heteroaryl ist; oder ein fluoriertes C₆-C₂₀-Aryl oder C₇₋C₂₀-Alkylaryl, wobei der Arylteil dieser Gruppen eine oder mehrere lineare oder verzweigte C₁-C₁₈-Alkyl-, C₁-C₁₈-Alkoxy-, C₂-C₁₀₋Alkenyl- oder C₃-C₁₅-Alkylalkenylgruppen als Substituenten tragen kann;
R⁴ Wasserstoff oder eine C₁-C₂₀-Gruppe ist;
R⁵, R⁶ identisch oder verschieden sind und jeweils Wasserstoff oder eine C₁-C₂₀-Gruppe sind.

6. Katalysatorsystem, umfassend eine oder mehrere Verbindungen der Formel (I), wie in Anspruch 1 oder 2 beansprucht, und einen oder mehrere Cokatalysatoren und/oder Träger.

7. Verwendung eines Katalysatorsystems, wie in Anspruch 6 beansprucht, für die Herstellung eines Polyolefins, insbesondere eines Copolymeren von verschiedenen Olefinen.

8. Verwendung einer Verbindung der Formel (I), wie in Anspruch 1 oder 2 beansprucht, für die Herstellung eines Polyolefins, insbesondere eines Copolymers von verschiedenen Olefinen.

9. Verwendung, wie in Anspruch 7 oder 8 beansprucht, für die Herstellung von Ethylen-Propylen-Copolymeren.

10. Verfahren zur Herstellung eines Polyolefins durch Polymerisieren von einem oder mehreren Olefinen in Gegenwart von einer oder mehreren Verbindungen der Formel (I), wie in Anspruch 1 oder 2 beansprucht.

## Revendications

1. Composé de métal de transition ayant la formule (I) 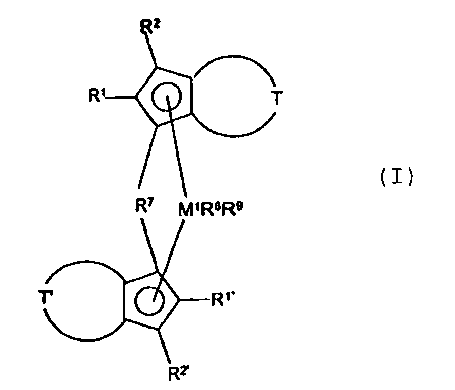
où 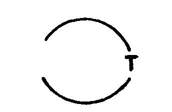
est 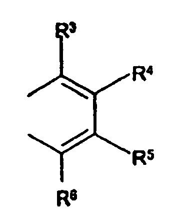
et 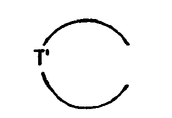
est 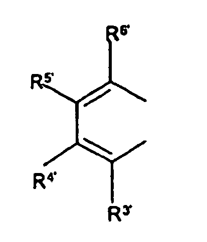
et
M¹ est du titane, du zirconium ou de l'hafnium,
R¹, R² sont identiques ou différents et sont chacun un groupe en C₁-C₂₀,
R^{1'},R^{2'} sont identiques ou différents, identiques ou différents de R¹ ou R² et sont chacun de l'hydrogène ou un groupe en C₁-C₂₀,
R³ est un groupe aryle en C₆-C₁₈ ou un hétéroaryle en C₁₄-C₁₈, ou un aryle en C₆-C₂₀ ou un alkylaryle en C₇-C₂₀ fluoré, où la partie aryle de ces groupes peut porter un ou plusieurs groupes linéaires ou ramifiés d'alkyle en C₁-C₁₈, alkoxy en C₁-C₁₈, un alkényle en C₂-C₁₀ ou alkylalkényle en C₃-C₁₅ en tant que substituants, ou R³ ensemble avec R⁴ forment un cycle monocyclique ou polycyclique qui peut à son tour être substitué,
R^{3'} est de l'hydrogène ou un groupe en C₁-C₄₀ ou R3' ensemble avec R4' forment un système de cycle monocyclique ou polycyclique qui peut à son tour être substitué,
R⁴, R^{4'} sont identiques ou différents et sont chacun de l'hydrogène ou un groupe en C₁-C₂₀,
R⁵, R^{5'}, R⁶ R^{6'} sont identiques ou différents et sont chacun de l'hydrogène ou un groupe en C₁-C₂₀,
R⁷ est un élément de structure de liaison entre deux radicaux indényle et est sélectionné parmi le groupe M²R¹⁰R¹¹, où M² est du silicium, du germanium, de l'étain ou du carbone et R¹⁰ et R¹¹ peuvent être identiques ou différents et sont chacun de l'hydrogène ou un groupe contenant un hydrocarbure en C₁-C₂₀,
R⁸, R⁹ peuvent être identiques ou différents et sont chacun un halogène, un alkyle en C₁-C₂₀ linéaire ou ramifié, un phénoxyde substitué ou non-substitué, ou R⁸ et R⁹ sont reliés l'un à l'autre et forment un système de cycle monocyclique ou polycyclique qui peut à son tour être substitué.

2. Composé de métal de transition selon la revendication 1, dans lequel
M¹ est du zirconium,
R¹ R² sont identiques ou différents et sont chacun un groupe alkyle en C₁-C₁₂,
R^{1'}, R²' sont identiques ou différents et sont chacun de l'hydrogène, du méthyle, de l'éthyle, du n-propyle, de l'isopropyle, du n-butyle, de l'isobutyle, du tert-butyle, du cyclopentyle ou du cyclohexyle,
R³, R^{3'} sont identiques ou différents et sont chacun un groupe aryle en C₆-C₁₈ ou deux radicaux R³ ensemble avec R⁴ et/ou R^{3'} ensemble avec R^{4'} peuvent former un système de cycle monocyclique ou polycyclique qui peut à son tour être substitué, et R^{3'} peut également être de l'hydrogène,
R⁴, _{R}⁴' sont identiques ou différents et sont chacun de l'hydrogène ou R⁴ ensemble avec R³ et/ou R^{4'} ensemble avec R^{3'} forment un système de cycle monocyclique ou polycyclique,
R ⁵ R^{5'}, R⁶, R^{6'} sont identiques ou différents et sont chacun de l'hydrogène, un alkyle en C₁-C₁₈ linéaire ou ramifié, un alkényle en C₂-C₁₀ ou un alkylalkényle en C₃-C₁₅, un aryle en C₆-C₂₀, un hétéroaryle en C₄-C₁₈, un alkylaryle en C₇-C₂₀, ou un alkyle en C₁-C₁₂ fluoré, un alkényle en C₂-C₁₀ fluoré, un aryle en C₆-C₂₀ fluoré ou un arylalkyle en C₇-C₂₀ fluoré,
R⁷ est un élément structurel de liaison SiR¹⁰R¹¹ et R¹⁰ et R¹¹ sont identiques ou différents et sont chacun un groupe contenant un hydrocarbure en C₁-C₂₀, et
R⁸, R⁹ sont chacun un chlore ou un méthyle.

3. Système de ligand ayant la formule (II) ou ses isomères à double liaison, 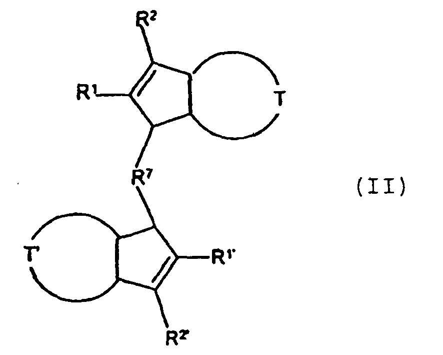
où les variables sont comme défini pour la formule (I) de la revendication 1.

4. Procédé pour préparer des ansa-métallocènes ayant la formule (I) tels que revendiqués selon la revendication 1, qui comporte les étapes suivantes :
a) réaction d'un 1-indanone ayant la formule (III) ou (III') avec un composé organométallique M³R²ₘHalₙ ou M³R^{2'}ₘHalₙ et élimination ultérieure pour former l'indène substitué ayant la formule (IV) ou (IV') 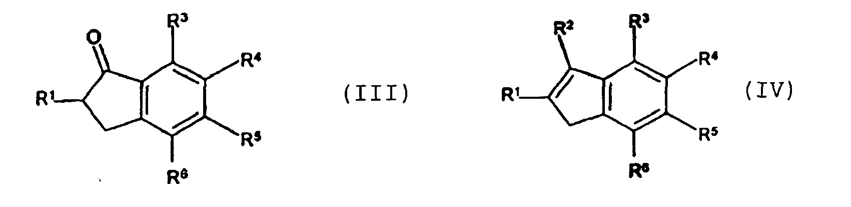 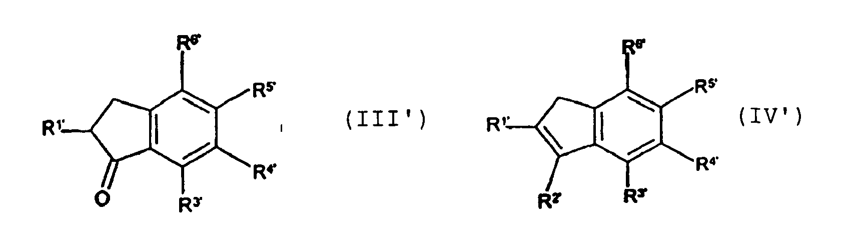 où les variables R¹, R^{1'}, R² R^{2'},R³, R^{3'}, R⁴, R^{4'}, R⁵, R^{5'}, R⁶ et R^{6'} sont comme définies pour la formule (I) de la revendication 1, M³ est un métal alcalin, un métal alcalinoterreux, de l'aluminium ou du titane, Hal est un halogène, m est un nombre entier et est égal ou supérieur à 1 et la somme de m+n correspond à la valence de M³,
b) déprotonation de l'indène substitué ayant la formule (IV) ou (IV') et réaction ultérieure de l'indène déprotoné avec des composés du type R⁷X₂ pour former des composés ayant la formule (V) ou (V') ou leurs isomères à double liaison, 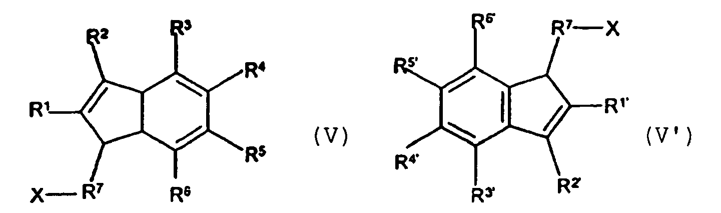
où X est Cl, Br, I ou O-tosyle et R⁷ est comme défini pour la formule (I) de la revendication 1 ;
c) réaction du composé ayant la formule (V) ou (V') avec un indène déprotoné supplémentaire qui a été obtenu par déprotonation de (IV) ou (IV') pour former le système de ligand de la formule (IIa) ou ses isomères à double liaison, 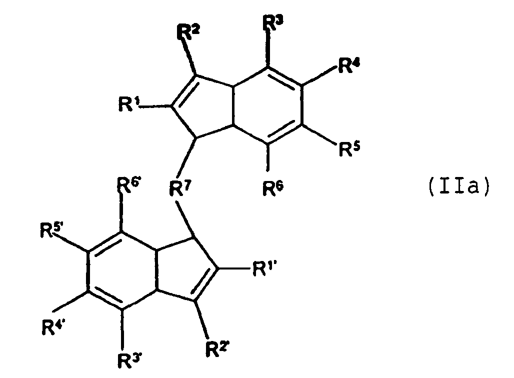
d) déprotonation du système de ligand ayant la formule (IIa) ou ses isomères à double liaison et réaction avec des composés du type X₂M¹R⁸R⁹ pour donner l'ansa-métallocène ayant la formule (I), où X est comme défini pour la formule (V) et M¹, R⁸ et R⁹ sont comme définis pour la formule (I) de la revendication 1.

5. Indène ayant la formule (IV) ou son isomère à double liaison, 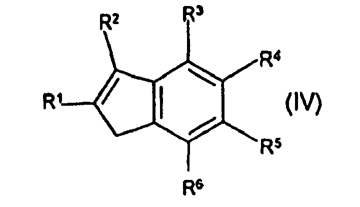
où
R¹, R² sont identiques ou différents et sont chacun un groupe en C₁-C₂₀,
R³ est un groupe aryle en C₆-C₁₈ ou un hétéroaryle en C₄-C₁₈, ou un aryle en C₆-C₂₀ ou alkylaryle en C₇-C₂₀ fluoré, où la partie aryle de ces groupes peut porter un ou plusieurs groupes alkyle en C₁-C₁₈, un alkoxy en C₁-C₁₈, un alkényle en C₂-C₁₀ ou alkylalkényle en C₃-C₁₅ linéaires ou ramifiés en tant que substituants,
R⁴ est un hydrogène ou un groupe en C₁-C₂₀,
R ⁵ R^{6'} sont identiques ou différents et sont chacun de l'hydrogène ou un groupe en C₁-C₂₀.

6. Système catalytique comportant un ou plusieurs composés ayant la formule (I) tels que revendiqué à la revendication 1 ou 2 et un ou plusieurs co-catalyseurs et/ou supports.

7. Utilisation d'un système catalytique selon la revendication 6 pour la préparation d'une polyoléfine, en particulier un copolymère de diverses oléfines.

8. Utilisation d'un composé ayant la formule (I) selon la revendication 1 ou 2 pour la préparation d'une polyoléfine, en particulier un copolymère de diverses oléfines.

9. Utilisation selon la revendication 7 ou 8 pour la préparation de copolymères d'éthylène-propylène.

10. Procédé pour préparer une polyoléfine par polymérisation d'une ou plusieurs oléfines en présence d'un ou plusieurs composés ayant la formule (I) tels que revendiqué à la revendication 1 ou 2.
